# EUROPÄISCHE PATENTSCHRIFT

(11) **EP 1 670 778 B1**
(45) Veröffentlichungstag und Bekanntmachung des Hinweises auf die Patenterteilung: **15.11.2006**
(21) Anmeldenummer: 04790276.2
(22) Anmeldetag: 06.10.2004
(51) Int. Cl.: C07D 311/76, C07D 237/32, C07D 231/56, C07D 209/46, C07D 239/74, C07D 215/38, A61K 31/416, A61K 31/4035, A61K 31/47, A61K 31/352, A61P 29/00

(54) **UMGELAGERTE PENTANOLE, EIN VERFAHREN ZU IHRER HERSTELLUNG UND IHRE VERWENDUNG ALS ENTZ NDUNGSHEMMER**
REARRANGED PENTANOLS, A METHOD FOR THE PRODUCTION THEREOF, AND THEIR USE AS ANTIPHLOGISTICS
PENTANOLS REARRANGES, UN PROCEDE POUR LEUR PRODUCTION ET LEUR UTILISATION EN TANT QU'ANTI-INFLAMMATOIRES

(30) Priorität: 08.10.2003 DE 10347385
(43) Veröffentlichungstag der Anmeldung: 21.06.2006
(73) Patentinhaber: Schering Aktiengesellschaft, 13353 Berlin (DE)
(72) Erfinder: REHWINKEL, Hartmut, 10961 Berlin (DE); BÄURLE, Stefan, 10245 Berlin (DE); BERGER, Markus, 13347 Berlin (DE); SCHMEES, Norbert, 13469 Berlin (DE); SCHÄCKE, Heike, 10115 Berlin (DE); KROLIKIEWICZ, Konrad, 12357 Berlin (DE); MENGEL, Anne, 10551 Berlin (DE); NGUYEN, Duy, 10179 Berlin (DE); JAROCH, Stefan, 10629 Berlin (DE); SKUBALLA, Werner, 13465 Berlin (DE)
(74) Vertreter: Seuss, Thomas
(86) Internationale Anmeldenummer: PCT/EP2004/011375
(87) Internationale Veröffentlichungsnummer: WO 2005/035518

(56) Entgegenhaltungen:
- EP-A- 1 375 517
- WO-A-00/32584
- WO-A-02/10143
- UOZUMI Y ET AL: "Asymmetric aza-Claisen rearrangement of allyl imidates catalyzed by homochiral cationic palladium(II) complexes" TETRAHEDRON: ASYMMETRY, ELSEVIER SCIENCE PUBLISHERS, AMSTERDAM, NL, Bd. 9, Nr. 6, 27. März 1998 (1998-03-27), Seiten 1065-1072, XP004131310 ISSN: 0957-4166

## Beschreibung

Die Erfindung betrifft Verbindungen der Formel I, ein Verfahren zu ihrer Herstellung und ihre Verwendung als Entzündungshemmer. Aus dem Stand der Technik DE 100 38 639 und WO02/10143 sind Entzündungshemmer der folgenden allgemeinen Formel 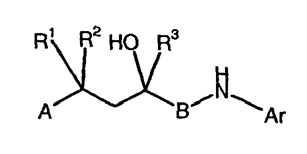 bekannt, wobei der Ar-Rest Phthalide, Thiophthalide, Benzoxazinone oder Phthalazinone umfaßt. Diese Verbindungen zeigen im Experiment Wirkdissoziationen zwischen antiinflammatorischen und unerwünschten metabolischen Wirkungen und sind den bisher beschriebenen, nichtsteroidalen Glucocorticoiden überlegen oder weisen zumindest eine ebenso gute Wirkung auf.

Die Selektivität der Verbindungen des Standes der Technik gegenüber den anderen Steroidrezeptoren ist jedoch noch verbesserungsbedürftig.

Daher war es Aufgabe der vorliegenden Erfindung, Verbindungen zur Verfügung zu stellen, deren Selektivität gegenüber den anderen Steroidrezeptoren verbessert ist.

Diese Aufgabe wird von den Verbindungen gemäß der Patentansprüche gelöst.

Die vorliegende Erfindung betrifft daher Verbindungen der allgemeinen Formel I 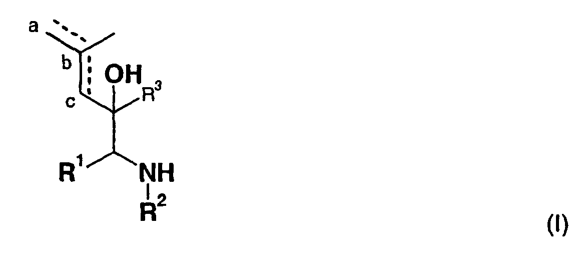 worin
- R¹: einen gegebenenfalls substituierten Phenylrest,
- R²: einen mono- oder bizyklisches aromatisches, teilaromatisches oder nicht aromatisches Ringsystem, das gegebenenfalls 1-3 Stickstoffatome, 1-2 Sauerstoffatome und/oder 1-2 Schwefelatome enthält und gegebenenfalls ein- oder mehrfach substituiert ist durch einen Rest ausgewählt aus der Gruppe Carbonyl, Halogen, Hydroxy, (C₁-C₅)-Alkyl, der gegebenenfalls substituiert sein kann durch 1-3 Hydroxygruppen, 1-3 (C₁-C₅)Alkoxygruppen und/oder 1-3 COOR⁶⁻Gruppen,
(C₁-C₅)Alkoxy, (C₁-C₅)-Alkylthio, (C₁-C₅)-Perfluoralkyl, Cyano, Nitro, oder zwei Substituenten zusammen eine Gruppe ausgewählt aus den Gruppen -O-(CH₂)ₙ-O-, -O-(CH₂)ₙ-CH₂-, -O-CH=CH-, -(CH₂)ₙ₊₂-, -NH- (CH₂)ₙ₊₁-, -N(C₁-C₃-alkyl)-(CH₂)ₙ₊₁-, -NH-N=CH-, bilden
wobei n = 1 oder 2 ist und die endständigen Sauerstoffatome und/oder Kohlenstoffatome und/oder Stickstoffatome mit direkt benachbarten Ring-Kohlenstoffatomen verknüpft sind, NR⁴R⁵
wobei R⁴ und R⁵ unabhängig voneinander Wasserstoff, C₁-C₅-Alkyl oder (CO)-C₁-C₅-Alkyl sein können, COOR⁶,
wobei R⁶ Wasserstoff oder eine C₁-C₅-Alkylgruppe bedeuten, (CO)NR⁷R⁸,
wobei R⁷ und R⁸ unabhängig voneinander Wasserstoff oder eine C₁-C₅₋Alkylgruppe bedeuten,
oder eine (C₁-C₅-Alkylen)-O-(CO)-(C₁-C₅)alkylgruppe
- R³: eine gegebenenfalls teilweise oder vollständig fluorierte C₁-C₃-Alkylgruppe,
worin der Phenylrest R¹ gegebenenfalls substituiert ist durch einen oder mehrere Reste aus der Gruppe C₁-C₅-Alkyl, C₁-C₅-Alkoxy, C₁-C₅-Alkylthio, C₁-C₅₋Perfluoralkyl, Halogen, Hydroxy, Cyano, Nitro, -O-(CH₂)ₙ-O-, -O-(CH₂)ₙ-CH₂-, -O-CH=CH-, -(CH₂)ₙ₊₂-, -NH-(CH₂)ₙ₊₁, N(C₁-C₃₋alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind, oder NR⁴R⁵, wobei R⁴ und R⁵ wie zuvor definiert sind,
die gestrichelte Linie eine Doppelbindung zwischen den Atomen a und b oder eine Doppelbindung zwischen den Atomen b und c oder nur eine Einfachbindung zwischen den Atomen a und b sowie b und c bedeutet, sowie deren Racemate oder getrennt vorliegende Stereoisomere, und gegebenenfalls deren physiologisch verträgliche Salze.

Ein weiter Gegenstand der Erfindung sind Stereoisomere der allgemeinen Formel I worin
- R¹: einen gegebenenfalls substituierten Phenylrest,
- R²: einen mono- oder bizyklisches aromatisches, teilaromatisches oder nicht aromatisches Ringsystem, das gegebenenfalls 1-3 Stickstoffatome, 1-2 Sauerstoffatome und/oder 1-2 Schwefelatome enthält und gegebenenfalls ein- oder mehrfach substituiert ist durch einen Rest ausgewählt aus der Gruppe Carbonyl, Halogen, Hydroxy, (C₁-C₅)-Alkyl, der gegebenenfalls substituiert sein kann durch 1-3 Hydroxygruppen,1-3 (C₁-C₅)Alkoxygruppen und/oder 1-3 COOR⁶⁻Gruppen,
(C₁-C₅)Alkoxy, (C₁-C₅)-Alkylthio, (C₁-C₅)-Perfluoralkyl, Cyano, Nitro, oder NR⁴R⁵
wobei R⁴ und R⁵ unabhängig voneinander Wasserstoff, C₁-C₅-Alkyl oder (CO)-C₁-C₅-Alkyl sein können, COOR⁶,
wobei R⁶ Wasserstoff oder eine C₁-C₅-Alkylgruppe bedeuten, (CO)NR⁷R⁸,
wobei R⁷ und R⁸ unabhängig voneinander Wasserstoff oder eine C₁-C₅₋Alkylgruppe bedeuten,
oder eine (C₁-C₅-Alkylen)-O-(CO)-(C₁-C₅)alkylgruppe
- R³: eine C₁-C₃-Alkylgruppe oder eine teilweise oder vollständig fluorierte C₁₋C₃-Alkylgruppe
bedeuten, worin der Phenylrest R¹ gegebenenfalls substituiert ist durch einen oder mehrere Reste aus der Gruppe C₁-C₅-Alkyl, C₁-C₅-Alkoxy, C₁-C₅-Alkylthio, C₁-C₅₋Perfluoralkyl, Halogen, Hydroxy, Cyano, Nitro, -O-(CH₂)ₙ-O-, -O-(CH₂)ₙ-CH₂-, -O-CH=CH-, -(CH₂)ₙ₊₂-, -NH-(CH₂)ₙ₊₁, N(C₁-C₃₋alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind, oder NR⁴R⁵, wobei R⁴ und R⁵ wie zuvor definiert sind,
die gestrichelte Linie eine Doppelbindung zwischen den Atomen a und b oder eine Doppelbindung zwischen den Atomen b und c oder nur eine Einfachbindung zwischen den Atomen a und b sowie b und c bedeutet, sowie deren Racemate oder getrennt vorliegenden Stereoisomeren, und gegebenenfalls deren physiologisch verträgliche Salze.

Ein weiterer Gegenstand der Erfindung sind Verbindungen der allgemeinen Formel I, worin der Phenylrest R¹ substituiert ist durch einen oder mehrere Reste aus der Gruppe C₁-C₅-Alkyl, C₁-C₅-Alkoxy, C₁-C₅-Alkylthio, C₁-C₅₋Perfluoralkyl, Halogen, Hydroxy, Cyano, Nitro,
-O-(CH₂)ₙ-O-, -O-(CH₂)ₙ-CH₂-, -O-CH=CH-, -(CH₂)ₙ₊₂-, -NH-(CH₂)ₙ₊₁, N(C₁-C₃₋alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁴R⁵,
wobei R⁴ und R⁵ unabhängig voneinander Wasserstoff, C₁-C₅-Alkyl oder (CO)-C₁-C₅-Alkyl sein können. Bevorzugt sind C₁-C₅-Alkyl, C₁-C₅-Alkoxy, Hydroxy und Halogen.

Bevorzugter Gegenstand der Erfindung sind Verbindungen, die als R¹ einen di- oder trisubstituierten Phenylrest enthalten.
Bevorzugte Substituenten für die Gruppe R¹ sind eine Alkanoylgruppe, C₁-C₅₋Alkyl, C₁-C₅-Alkoxy, Hydroxy, Halogen, Cyano, -O-(CH₂)ₙ-O-, -O-(CH₂)ₙ-CH₂-, - O-CH=CH-, -(CH₂)ₙ₊₂-, besonders bevorzugte Substituenten für die Gruppe R¹ sind eine Alkanoylgruppe, C₁-C₅-Alkyl, C₁-C₅-Alkoxy, Hydroxy, Halogen und Cyano.

Mit Alkanoyl ist ein Rest (C₁-C₅)Alkyl-(CO)- gemeint.

Ein Gegenstand der Erfindung sind Verbindungen der allgemeinen Formel 1, worin R² ein mono-oder bizyklisches aromatisches oder teilaromatisches heterozyklisches Ringsystem, insbesondere ein bizyklisches Ringsystem bedeutet.

Mit der Definition mono- oder bizyklisches aromatisches, teilaromatisches oder nicht aromatisches Ringsystem, das gegebenenfalls 1-3 Stickstoffatome und/oder 1-2 Sauerstoffatome und/oder 1-2 Schwefelatome enthält werden aromatische Ringsysteme, bizyklische Ringsysteme die nur einen aromatischen Ring enthalten und aliphatische Ringsysteme umfaßt, die 1-7 Heteroatome enthalten können oder auch kein Heteroatom enthalten.

Ein Gegenstand der Erfindung sind Verbindungen der allgemeinen Formel I, worin R² eine über eine beliebige Position verknüpfte gegebenenfalls substituierte Phthalidyl-, Isoindolyl-, Dihydroindolyl-, Dihydroisoindolyl-, Dihydroisochinolinyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl-, 1,7- oder 1,8-Naphthyridinyl-, Dihydroindolonyl-, Dihydroisoindolonyl-, Benzimidazol- oder Indolylgruppe bedeutet, insbesondere wenn diese heterozyklischen Systeme substituiert sind , bevorzugt, wenn sie mit 0 bis 3 gleichen oder verschiedenen Resten aus der Gruppe C₁-C₃-Alkyl, Hydroxy, Carbonyl oder Halogen substituiert sind, besonders bevorzugt, wenn sie mit Methyl, Chlor oder Fluor substituiert sind.

Ein weiterer Gegenstand der Erfindung sind Verbindungen der allgemeinen Formel I, worin R² eine über eine beliebige Position verknüpfte Phthalidyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl, 1,7- oder 1,8-Naphthyridinyl, Dihydroindolonyl-, Dihydroisoindolonyl-, Benzimidazol- oder Indolylgruppe bedeutet.

Ein Gegenstand der Erfindung sind Verbindungen der Formel I, die für R² einen Cumarinyl- oder Isocumarinylrest, insbesondere den Isocumarinylrest tragen, der gegebenenfalls substituiert sein kann mit 0 bis 3 gleichen oder verschiedenen Resten aus der Gruppe C₁-C₃-Alkyl, Hydroxy, Carbonyl oder Halogen, insbesondere mit Methyl, Chlor oder Fluor, besonders die Verbindung des Beispiels 9.

R² kann ein oder mehrfach substitutiert sein mit einem Rest aus der Gruppe Carbonyl, Halogen, Hydroxy, (C₁-C₅)-Alkyl, (C₁-C₅)Alkoxy, (C₁-C₅)-Alkylthio, (C₁₋C₅)-Perfluoralkyl, Cyano, Nitro, NR⁴R⁵, COOR⁶, (CO)NR⁷R⁸ oder eine (C₁-C₅₋Alkylen)-O-(CO)-(C₁-C₅)alkylgruppe,
bevorzugt aus der Gruppe C₁-C₃-Alkyl, C₁-C₃-Alkoxy, Hydroxy, Halogen, Carbonyl; vorzugsweise mit Methyl, Chlor oder Fluor. Die Substituenten können gleich oder verschieden sein.
Der Substituent Carbonyl für eine Gruppe R² ist so zu verstehen, dass das Carbonylkohlenstoffatom ein Ringkohlenstoffatom ist, an das ein Sauerstoffatom doppelt gebunden ist.

Ein Gegenstand der Erfindung sind Verbindungen der allgemeinen Formel l, worin der Rest R² mit keinem, einem oder mehreren gleichen oder verschiedenen Resten aus der Gruppe C₁-C₃-Alkyl, C₁-C₃-Alkoxy, Hydroxy, Halogen, Carbonyl, bevorzugt mit keinem oder einem oder mehreren gleichen oder verschiedenen Reste aus der Gruppe C₁-C₃-Alkyl, Hydroxy, Carbonyl oder Halogen substituiert ist, insbesondere durch einen oder mehrere gleiche oder verschiedene Reste aus der Gruppe Methyl, Chlor oder Fluor, besonders durch Methyl, Chlor oder Fluor substituiert ist.

Das Stickstoffatom im Indazol, Chinolon, Isochinolon und Phthalazin des Restes R² des allgemeinen Anspruches 1 kann auch alkyliert sein mit einer C₁-C₃₋Alkylgruppe.

Ein weiterer Gegenstand der Erfindung sind Verbindungen der allgemeinen Formel I, worin R² ein über eine beliebige Position verknüpftes monozyklisches 5- oder 6-gliedriges heterozyklisches Ringsystem wie z.B. Furan oder Thiophen bedeutet.

Ein weiterer Gegenstand der Erfindung sind Verbindungen der allgemeinen Formel I, worin R² einen gegebenenfalls substituierten Phenylring oder Naphthylring bedeutet.
Als Substituenten sind dieselben geeignet, die bereits für den Fall, dass R¹ Phenyl bedeutet, offenbart sind.

Mit einem teilaromatischen Ringsystem sind bizyklische Systeme gemeint, die einen aromatischen und einen nicht aromatischen Ring enthalten wie z.B. Benzoxazinone oder Dihydroindolon.

Ein besonderer Gegenstand der Erfindung sind Verbindungen gemäß Anspruch 1, worin R³ Trifluormethyl oder Pentafluorethyl bedeutet. Ein bevorzugter Gegenstand der Erfindung sind Verbindungen der allgmeinen Formel l, worin
R¹ eine Phenylgruppe, die gegebenenfalls substituiert ist mit O-3 gleichen oder verschiedenen Substituenten ausgewählt aus der Gruppe Carbonyl, C₁-C₃₋Alkoxy, Hydroxy, und Halogen, insbesondere Carbonyl, Methoxy, Hydroxy, Fluor, Chlor, Brom,
R² eine Dihydroisoindolonyl, Isochinolonyl, Chinazolinyl, Indazolyl, Cumarinyl-, Isocumarinyl, insbesondere Dihydroisoindolonyl, Isochinolonyl, Chinazolinyl, Indazolyl, Isocumarinyl, Phthalazinyl, Chinolonylgruppe , die gegebenenfalls substituiert sein kann mit 0-2 Substituenten ausgewählt aus der Gruppe Carbonyl, C₁-C₃-Alkyl und Halogen, insbesondere Methyl und Fluor und
R³ CF₃ oder C₂F₅, insbesondere CF₃ bedeutet.

Die Erfindung betrifft weiterhin die Verwendung der Verbindungen der allgemeinen Formel I zur Herstellung von Arzneimitteln sowie ihre Verwendung zur Herstellung von Arzneimitteln zur Behandlung von entzündlichen Erkrankungen.

Die C₁-C₅-Alkylgruppen können geradkettig oder verzweigt sein und für eine Methyl-, Ethyl-, n-Propyl-, iso-Propyl-, n-Butyl, iso-Butyl, tert.-Butyl- oder n-Pentyl-, 2,2-Dimethylpropyl-, 2-Methylbutyl- oder 3-Methylbutylgruppe stehen. Eine Methyl- oder Ethylgruppe ist bevorzugt. Sie können gegebenenfalls substituiert sein durch 1-3 Hydroxy- 1-3-C₁-C₅-Alkoxy- und/oder 1-3- COOR⁶ Gruppen. Bevorzugt sind Hydroxygruppen.
COOR⁶ kann die freie Säure oder ein C₁-C₅-Alkylester sein, wobei der Alkylrest wie bereits oben beschrieben definiert ist.

Die C₁-C₅-Alkoxygruppen in R¹ und R² können geradkettig oder verzweigt sein und für eine Methoxy-, Ethoxy-, n-Propoxy-, iso-Propoxy-, n-Butoxy, iso-Butoxy, tert.-Butoxy- oder n-Pentoxy-, 2,2-Dimethylpropoxy-, 2-Methylbutoxy- oder 3-Methylbutoxygruppe stehen. Eine Methoxy- oder Ethoxygruppe ist bevorzugt.

Die C₁-C₅-Alkylthiogruppen können geradkettig oder verzweigt sein und für eine Methylthio-, Ethylthio-, n-Propylthio-, iso-Propylthio-, n-Butylthio-, iso-Butylthio-, tert.-Butylthio- oder n-Pentylthio-, 2,2-Dimethylpropylthio-, 2-Methylbutylthio- oder 3-Methylbutylthiogruppe stehen. Eine Methylthio- oder Ethylthiogruppe ist bevorzugt.

Für eine teilweise oder vollständig fluorierte C₁-C₃-Alkylgruppe kommen die teilweise oder vollständig fluorierten folgenden Gruppen in Betracht: Fluormethyl, Difluormethyl, Trifluormethyl, Fluorethyl, 1,1-Difluorethyl, 1,2-Difluorethyl, 1,1,1-Trifluorethyl, Tetrafluorethyl, Pentafluorethyl. Von diesen bevorzugt sind die Trifluormethyl- oder die Pentafluorethylgruppe.

Die Bezeichnung Halogenatom oder Halogen bedeutet ein Fluor-, Chlor-, Brom- oder lodatom. Bevorzugt ist ein Fluor-, Chlor- oder Bromatom.

Die NR⁴R⁵-Gruppe kann beispielsweise NH₂, N(H)CH₃, N(CH₃)₂, N(H)(CO)CH₃, N(CH₃)(CO)CH₃, N[(CO)CH₃]₂, N(H)CO₂CH₃, N(CH₃)CO₂CH₃, N(CO₂CH₃)₂, bedeuten.

Die Doppelbindung in der allgemeinen Formel I kann zwischen den Atomen a und b oder zwischen den Atomen b und c liegen.

Auch Verbindungen, die weder zwischen den Atomen a und b noch zwischen den Atomen b und c eine Doppelbindung aufweisen sind Gegenstand der Erfindung.

Die erfindungsgemäßen Verbindungen der allgemeinen Formel I können durch das Vorhandensein von Asymmetriezentren als Stereoisomere vorliegen. Gegenstand der vorliegenden Erfindung sind alle möglichen Diastereomere, sowohl als Racemate als auch in enantiomerenreiner Form.

Die erfindungsgemäßen Verbindungen können auch in Form von Salzen mit physiologisch verträglichen Anionen vorliegen, beispielsweise in der Form des Hydrochlorides, Sulfates, Nitrates, Phosphates, Pivalates, Maleates, Fumarates, Tartrates, Benzoates, Mesylates, Citrates oder Succinates.

Die Verbindungen können durch im folgendenden beschriebenes Verfahren hergestellt werden.

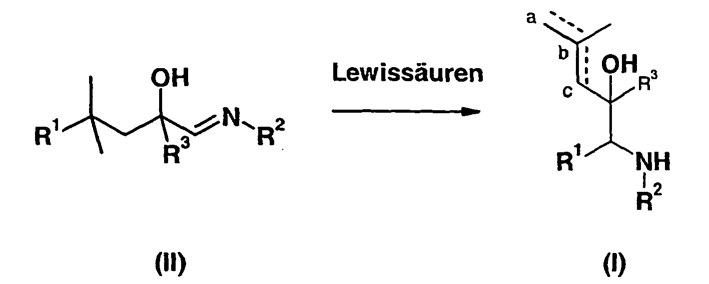

Die in dem Anmeldungstext beschriebenen Verbindungen entstehen bei der Reaktion der entsprechenden Imine mit Lewissäuren, besonders bei der Reaktion mit Bortribromid in einem organischen Lösungsmittel, bevorzugt in einem polaren Lösungsmittel, beispielsweise Dichlormethan und nachfolgender üblicher Aufreinigung. Die Reaktion erfolgt in einem Temperaturbereich von - 70°C bis +30°C (bevorzugt -30°C bis +30°C). Die Imine wiederum werden gebildet, indem die entsprechenden beschriebenen Aldehyde mit den gewünschten Aminen in üblicher Weise umgesetzt werden. Dieses geschieht durch Umsetzung mit Titanaten oder durch Reaktion im sauren Milieu oder unter annähernd neutralen Bedingungen (beispielsweise THF/Alkohol), entweder durch Rühren bei Raumtemperatur oder durch Kochen am Wasserabscheider.

Wenn die erfindungsgemäßen Verbindungen als racemische Gemische vorliegen, können sie nach dem Fachmann geläufigen Methoden der Racemattrennung in die reinen, optisch aktiven Formen aufgetrennt werden. Beispielsweise lassen sich die racemischen Gemische durch Chromatographie an einem selbst optisch aktiven Trägermaterial (CHIRALPAK AD^{®}) in die reinen Isomere trennen. Es ist auch möglich, die freie Hydroxygruppe in einer racemischen Verbindung der allgemeinen Formel I mit einer optisch aktiven Säure zu verestern und die erhaltenen diastereoisomeren Ester durch fraktionierte Kristallisation oder chromatographisch zu trennen und die getrennten Ester jeweils zu den optisch reinen Isomeren zu verseifen. Als optisch aktive Säure kann beispielsweise Mandelsäure, Camphersulfonsäure oder Weinsäure verwendet werden.

Die Bindung der Substanzen an den Glucocorticoid-Rezeptor (GR) und weitere Steroidhormon-Rezeptoren (Mineralcorticoid-Rezeptor (MR), Progesteron-Rezeptor (PR) und Androgen-Rezeptor (AR)) wird mit Hilfe rekombinant hergestellter Rezeptoren überprüft. Cytosolpräparationen von Sf9 Zellen, die mit rekombinanten Baculoviren, die für den GR kodieren, infiziert worden waren, werden für die Bindungsuntersuchungen eingesetzt. Im Vergleich zur Bezugssubstanz [³H]-Dexamethason zeigen die Substanzen eine hohe bis sehr hohe Affinität zum GR. So wurde für die Verbindung aus Beispiel 3 IC₅₀(GR) = 64 nM gemessen.

Als wesentlicher, molekularer Mechanismus für die anti-inflammatorische Wirkung von Glucocorticoiden wird die durch den GR vermittelte Hemmung der Transkription von Cytokinen, Adhäsionsmolekülen, Enzymen und anderer pro - inflammatorischen Faktoren angesehen. Diese Hemmung wird durch eine Interaktion des GR mit anderen Transkriptionsfaktoren, z.B. AP-1 und NFkappa-B, bewirkt (zur Übersicht siehe Cato ACB and Wade E, BioEssays 18, 371-378 1996).

Die erfindungsgemäßen Verbindungen der allgemeinen Formel I hemmen die durch Lipopolysaccharid (LPS) ausgelöste Sekretion des Cytokins IL-8 in der menschlichen Monozytenzelline THP-1. Die Konzentration der Cytokine wurde im Überstand mittels kommerziell erhältlicher ELISA-Kits bestimmt. Die Verbindung aus Beispiel 3 zeigte eine Inhibition IC₅₀(IL8) = 25 nM.

Die anti - inflammatorische Wirkung der Verbindungen der allgemeinen Formel I wurde im Tierexperiment durch Testen in der Crotonöl - induzierten Entzündung in der Ratte und der Maus getestet (J. Exp. Med. (1995), 182, 99-108). Hierzu wurde den Tieren Crotonöl in ethanolischer Lösung topisch auf die Ohren appliziert. Die Testsubstanzen wurden gleichzeitig oder zwei Stunden vor dem Crotonöl ebenfalls topisch oder systemisch appliziert. Nach 16-24 Stunden wurden das Ohrgewicht als Maß für das entzündliche Ödem, die Peroxidaseaktivität als Maß für die Einwanderungen von Granulozyten und die Elastaseaktivität als Maß für die Einwanderung von neutrophilen Granulozyten gemessen. Die Verbindungen der allgemeinen Formel I hemmen in diesem Test sowohl nach topischer, als auch nach systemischer Applikation die drei oben genannten Entzündungsparameter.

Eine der häufigsten unerwünschten Wirkungen einer Glucocorticoid - Therapie ist der sogenannte "Steroiddiabetes" [vgl. Hatz, HJ, Glucocorticoide: Immunologische Grundlagen, Pharmakologie und Therapierichtlinien, Wissenschafliche Verlagsgesellschaft mbH, Stuttgart, 1998]. Ursache hierfür ist die Stimulation der Gluconeogenese in der Leber durch Induktion der hierfür verantwortlichen Enzyme und durch freie Aminosäuren, die aus dem Abbau von Proteinen (katabole Wirkung der Glucocorticoide) entstehen. Ein Schlüsselenzym des katabolen Stoffwechsels in der Leber ist die Tyrosinaminotranferase (TAT). Die Aktivität dieses Enzyms kann photometrisch aus Leberhomogenaten bestimmt werden und stellt ein gutes Maß für die unerwünschten metabolischen Wirkungen der Glucocorticoide dar. Zur Messung der TAT - Induktion werden die Tiere 8 Stunden nach Gabe der Testsubstanzen getötet, die Leber entnommen und die TAT - Aktivität im Homogenat gemessen. Die Verbindungen der allgemeinen Formel I induzieren in diesem Test in Dosen, in denen sie anti - inflammatorisch wirksam sind, nicht oder nur in geringem Maße die Tyrosinaminotransferase.

Aufgrund ihrer anti-inflammatorischen und zusätzlichen anti-allergischen, immunsuppressiven und anti-proliferativen Wirkung können die erfindungsgemäßen Verbindungen der allgemeinen Formel I als Medikamente zur Behandlung oder Prophylaxe folgender Krankheitszustände bei Säugetieren und Menschen Verwendung finden: Dabei steht der Begriff "ERKRANKUNG" für die folgenden Indikationen:
(i) Lungenerkrankungen, die mit entzündlichen, allergischen und / oder proliferativen Prozessen einhergehen:
   - Chronisch obstruktive Lungenerkrankungen jeglicher Genese, vor allem Asthma bronchiale
   - Bronchitis unterschiedlicher Genese
   - Alle Formen der restriktiven Lungenerkrankungen, vor allem allergische Alveolitis,
   - Alle Formen des Lungenödems, vor allem toxisches Lungenödem
   - Sarkoidosen und Granulomatosen, insbesondere Morbus Boeck
(ii) Rheumatische Erkrankungen / Autoimmunerkrankungen / Gelenkerkrankungen, die mit entzündlichen, allergischen und / oder proliferativen Prozessen einhergehen:
   - Alle Formen rheumatischer Erkrankungen, insbesondere rheumatoide Arthritis, akutes rheumatisches Fieber, Polymyalgia rheumatica
   - Reaktive Arthritis
   - Entzündliche Weichteilerkrankungen sonstiger Genese
   - Arthritische Symptome bei degenerativen Gelenkerkrankungen (Arthrosen)
   - Traumatische Arthritiden
   - Kollagenosen jeglicher Genese, z.B. systemischer Lupus erythematodes, Sklerodermie, Polymyositis, Dermatomyositis- Sjögren-Syndrom, Still-Syndrom, Felty-Syndrom
(iii) Allergien, die mit entzündlichen, und / oder proliferativen Prozessen einhergehen:
   - Alle Formen allergischer Reaktionen, z.B. Quincke Ödem, Heuschnupfen, Insektenstich, allergische Reaktionen auf Arzneimittel, Blutderivate, Kontrastmittel etc., Anaphylaktischer Schock, Urtikaria, Kontakdermatitis
(iv) Gefäßentzündungen (Vaskulitiden)
   - Panarteriitis nodosa, Arteriitis temporalis, Erythema nodosum
(v) Dermatologische Erkrankungen, die mit entzündlichen, allergischen und / oder proliferativen Prozessen einhergehen:
   - Atopische Dermatitis (vor allem bei Kindern)
   - Psoriasis
   - Pityriasis rubra pilaris
   - Erythematöse Erkrankungen, ausgelöst durch unterschiedlichen Noxen, z.B. Strahlen, Chemikalien, Verbrennungen etc.
   - Bullöse Dermatosen
   - Erkrankungen des lichenoiden Formenkreises,
   - Pruritus (z. B. allergischer Genese)
   - Seborrhoisches Ekzem
   - Rosacea
   - Pemphigus vulgaris
   - Erythema exsudativum multiforme
   - Balanitis
   - Vulvitis
   - Haarausfall wie Alopecia areata
   - Cutane T - Zell - Lymphome
(vi) Nierenerkrankungen, die mit entzündlichen, allergischen und / oder proliferativen Prozessen einhergehen:
   - Nephrotisches Syndrom
   - Alle Nephritiden
(vii) Lebererkrankungen, die mit entzündlichen, allergischen und /oder proliferativen Prozessen einhergehen:
   - akuter Leberzellzerfall
   - akute Hepatitis unterschiedlicher Genese, z.B. viral, toxisch, arzneimittelinduziert
   - chronisch aggressive und / oder chronisch intermittierende Hepatitis
(viii) Gastrointestinale Erkrankungen, die mit entzündlichen, allergischen und / oder proliferativen Prozessen einhergehen:
   - regionale Enteritis (Morbus Crohn)
   - Colitis Ulcerosa
   - Gastritis
   - Refluxoesophagitis
   - Gastroenteritiden anderer Genese, z.B. einheimische Sprue
(ix) Proktologische Erkrankungen, die mit entzündlichen, allergischen und / oder proliferativen Prozessen einhergehen:
   - Analekzem
   - Fissuren
   - Hämorrhoiden
   - idiopathische Proktitis
(x) Augenerkrankungen, die mit entzündlichen, allergischen und /oder proliferativen Prozessen einhergehen:
   - allergische Keratitis, Uveitis, Iritis,
   - Konjunktivitis
   - Blepharitis
   - Neuritis nervi optici
   - Chorioditis
   - Ophtalmia sympathica
(xi) Erkrankungen des Hals-Nasen-Ohren-Bereiches, die mit entzündlichen, allergischen und / oder proliferativen Prozessen einhergehen:
   - allergische Rhinitis, Heuschnupfen
   - Otitis externa, z.B. bedingt durch Kontaktexem, Infektion etc.
   - Otitis media
(xii) Neurologische Erkrankungen, die mit entzündlichen, allergischen und / oder proliferativen Prozessen einhergehen:
   - Hirnödem, vor allem Tumor-bedingtes Hirnödem
   - Multiple Sklerose
   - akute Encephalomyelitis
   - Meningitis
   - verschieden Formen von Krampfanfällen, z.B. BNS-Krämpfe
(xiii) Bluterkrankungen, die mit entzündlichen, allergischen und / oder proliferativen Prozessen einhergehen:
   - Erworbene hämolytische Anämie
   - Idopathische Thrombocytopenia
(xiv) Tumorerkrankungen, die mit entzündlichen, allergischen und / oder proliferativen Prozessen einhergehen:
   - Akute lymphatische Leukämie
   - Maligne Lymphome
   - Lymphogranulomatosen
   - Lymphosarkome
   - Ausgedehnte Metastasierungen, vor allem bei Mamma- Bronchial- und Prostatakarzinom
(xv) Endokrine Erkrankungen, die mit entzündlichen, allergischen und / oder proliferativen Prozessen einhergehen:
   - Endokrine Orbitopathie
   - Thyreotoxische Krise
   - Thyreoiditis de Quervain
   - Hashimoto Thyreoiditis
   - Morbus Basedow
(xvi) Organ- und Gewebstransplantationen , Graft-versus-host-disease(xvii) Schwere Schockzustände, z.B anaphylaktischer Schock , systemic inflammatory response syndrome (SIRS)
(xviii) Substitutionstherapie bei:
   - angeborene primäre Nebenniereninsuffizienz, z.B. kongenitales adrenogenitales Syndrom
   - erworbene primäre Nebenniereninsuffizienz, z.B. Morbus Addison, autoimmune Adrenalitits, postinfektiös, Tumoren, Metastasen etc.
   - angeboren sekundäre Nebeniereninsuffizienz, z.B. kongenitaler Hypopitutitarismus
   - erworbene sekundäre Nebenniereninsuffizienz, z.B. postinfektiös, Tumoren etc.
(xix) Emesis, die mit entzündlichen, allergischen und / oder proliferativen Prozessen einhergehen:
   - z.B. in Kombination mit einem 5-HT3-Antagonisten bei Zytostika - bedingten Erbrechen.
(xx) Schmerzen bei entzündlicher Genese, z.B. Lumbago

Darüber hinaus können die erfindungsgemäßen Verbindungen der allgemeinen Formel I zur Therapie und Prophylaxe weiterer oben nicht genannter Krankheitszustände eingesetzt werden, für die heute synthetische Glucocorticoide verwendet werden (siehe dazu Hatz, HJ, Glucocorticoide: Immunologische Grundlagen, Pharmakologie und Therapierichtlinien, Wissenschafliche Verlagsgesellschaft mbH, Stuttgart, 1998).

Alle zuvor genannten Indikationen (i) bis (xx) sind ausführlich beschrieben in Hatz, HJ**,** Glucocorticoide: Immunologische Grundlagen, Pharmakologie und Therapierichtlinien, Wissenschafliche Verlagsgesellschaft mbH, Stuttgart, 1998.

Für die therapeutische Wirkungen bei den oben genannten Krankheitszuständen ist die geeignete Dosis unterschiedlich und hängt beispielsweise von der Wirkstärke der Verbindung der allgemeinen Formel I, dem Wirt, der Art der Verabreichung und der Art und der Schwere der zu behandelnden Zustände, sowie der Verwendung als Prophylaktikum oder Therapeutikum ab.

Die Erfindung liefert weiterhin
(i) die Verwendung eines der erfindungsgemäßen Verbindung gemäß Formel I oder deren Gemisch zur Herstellung eines Medikaments zur Behandlung von einer ERKRANKUNG;
(ii) ein Verfahren zur Behandlung von einer ERKRANKUNG, welches Verfahren eine Verabreichung einer Verbindungsmenge gemäß der Erfindung umfaßt, wobei die Menge die Krankheit unterdrückt, und wobei die Verbindungsmenge einem Patienten gegeben wird, der ein solches Medikament benötigt;
(iii) eine pharmazeutische Zusammensetzung zur Behandlung von einer ERKRANKUNG, welche Behandlung eines der erfindungsgemäßen Verbindungen oder deren Gemisch und wenigstens einen pharmazeutischen Hilfs- und/oder Trägerstoff umfaßt.

Im allgemeinen sind bei Tieren zufriedenstellende Resultate zu erwarten, wenn die täglichen Dosen einen Bereich von 1 µg bis 100.000 µg der erfindungsgemäßen Verbindung pro kg Körpergewicht umfassen. Bei größeren Säugetieren, beispielsweise dem Menschen, liegt eine empfohlene tägliche Dosis im Bereich von 1 µg bis 100.000 µg pro kg Körpergewicht. Bevorzugt ist eine Dosis von 10 bis 30.000 µg pro kg Körpergewicht, mehr bevorzugt eine Dosis von 10 bis 10.000 µg pro kg Körpergewicht. Zum Beispiel wird diese Dosis zweckmäßigerweise mehrmals täglich verabreicht. Zur Behandlung eines akuten Schocks (z.B. anaphylaktischer Schock) können Einzeldosen gegeben werden, die deutlich über den oben genannten Dosen liegen.

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmittein, Verdünnungsmitteln, Geschmackskorrigentien, Färbemitteln usw., verarbeitet und in die gewünschte Applikationsform überführt. Dabei ist auf Remington's Pharmaceutical Science, 15th ed. Mack Publishing Company, East Pennsylvania (1980) hinzuweisen.

Für die orale Applikation kommen insbesondere Tabletten, Dragees, Kapseln, Pillen, Pulver, Granulate, Pastillen, Suspensionen, Emulsionen oder Lösungen in Frage.

Für die parenterale Applikation sind Injektion- und Infusionszubereitungen möglich.

Für die intraartikulären Injektion können entsprechend zubereitete Kristallsuspensionen verwendet werden.

Für die intramuskuläre Injektion können wässrige und ölige Injektionslösungen oder Suspensionen und entprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen, als auch zur lokalen Therapie verwendet werden.

Zur pulmonalen Applikation der neuen Verbindungen können diese in Form von Aerosolen und Inhalaten verwendet werden.

Für die lokale Anwendung an Augen, äußerem Gehörgang, Mittelohr, Nasenhöhle und Nasennebenhöhlen können die neuen Verbindungen als Tropfen, Salben und Tinkturen in entsprechenden pharmazeutischen Zubereitungen verwendet werden.

Für die topische Auftragung sind Formulierungen in Gelen, Salben, Fettsalben, Cremes, Pasten, Puder, Milch und Tinkturen möglich. Die Dosierung der Verbindungen der allgemeinen Formel I sollte in diesen Zubereitungen 0.01% - 20% betragen, um eine ausreichende pharmakologische Wirkung zu erzielen.

Die Erfindung umfaßt ebenfalls die erfindungsgemäßen Verbindungen der allgemeinen Formel I als therapeutischen Wirkstoff.
Weiterhin gehört zur Erfindung die erfindungsgemäßen Verbindungen der allgemeinen Formel I ais therapeutischen Wirkstoff zusammen mit pharmazeutisch verträglichen und annehmbaren Hilfsstoffen und Trägerstoffen. Ebenfalls umfaßt die Erfindung eine pharmazeutische Zusammensetzung, die eine der pharmazeutisch aktiven, erfindungsgemäßen Verbindungen oder deren Gemisch oder deren pharmazeutisch verträgliches Salz und ein pharmazeutisch verträgliches Salz oder pharmazeutisch verträgliche Hilfsstoffe und Trägerstoffe enthält.

### Experimenteller Teil

### Beispiel 1

### (rac.) 4-{[1-(4-Chlor-2-methoxyphenyl)-2-hydroxy-4-methyl-2-(trifluormethyl)-pent-4-en-1-yl]amino}-6-fluor-2,3-dihydroisoindol-1-on

### 4-Amino-6-fluor-2,3-dihydroisoindol-1-on

### 2-Methyl-5-fluor-3-nitrobenzoesäure

116 ml Schwefelsäure werden vorgelegt und bei -15°C portionsweise mit 14,70 g (95,37 mmol) 5-Fluor-2-methylbenzoesäure versetzt. Zu dieser Mischung wird ein Gemisch Nitriersäure (4,79 ml rauchende Salpetersäure und 21,8 ml conc. Schwefelsäure) zugetropft, und zwar bei -15 bis -10°C während eines Zeitraums von 90 Minuten. Nach dreistündigem Nachrühren wird die Reaktionsmischung auf Eiswasser gegossen und ca. eine halbe Stunde kräftig gerührt. Das ausgefallene Kristallisat wird abgesaugt, mit Wasser neutralgewaschen und getrocknet. Die Ausbeute beträgt 8,56 g (45,1%) eines Gemisches verschiedener Regioisomere und Beiprodukte. Dieses Gemisch wird so in die nächste Stufe (Veresterung) eingesetzt und auf dieser Stufe gereinigt.

### 2-Methyl-5-fluor-3-nitrobenzoesäuremethylester

8,56 g (42,99 mmol) 2-Methyl-5-fluor-3-nitrobenzoesäure werden in 76 ml N,N-Dimethylformamid gegeben und mit 9,15 g (64,48 mmol) Methyiodid und 8,91 g (64,48 mmol) Kaliumcarbonat versetzt. Nach 65 Stunden Rühren bei Raumtemperatur wird die Reaktionsmischung auf Eiswasser gegeben und mehrfach mit Ethylacetat extrahiert. Die vereinigten organischen Extrakte werden mit Wasser und Sole gewaschen. Nach dem Trocknen (Natriumsulfat), wird vom Trockenmittel abgesaugt und das Lösungsmittel abrotiert. Mehrfache Chromatographie an Kieselgel (Laufmittel Ethylacetat/Hexan) ergibt die gewünschte Verbindung, und zwar in einer Ausbeute von 25,9% (2,37 g).
¹H-NMR (300 MHz, CDCl₃): δ = 2.60 (3H), 3.96 (3H), 7.61 (1 H), 7.77 (1 H).

### 2-(Brommethyl)-5-fluor-3-nitrobenzoesäuremethylester

2,37 g (11,12 mmol) 5-Fluor-2-methyl-3-nitrobenzoesäuremethylester werden in 35 ml Tetrachlorkohlenstoff gegeben, mit 2,24 Gramm (12,24 mmol) N-Bromsuccinimid und 5,4 mg Benzoylperoxid versetzt. Nach viertägigem Kochen am Rückfluß wird nach dem Abkühlen das Succinimid abgesaugt (Glasfaserfilter) und anschließend das Filtrat zur Trockene einrotiert. Chromatographie am Flashmaster ergibt 2,47 g (75,9%) der gewünschten Verbindung.
¹H-NMR (300 MHz, CDCl₃): δ = 4.01 (3H), 5.13 (2H), 7.72 (1 H), 7.87 (1 H).

### 2-(Azidomethyl)-5-fluor-3-nitrobenzoesäuremethylester

2,47 g (8,46 mmol) 2-(Brommethyl)-5-fluor-3-nitrobenzoesäuremethylester werde mit 8,3 ml N,N-Dimethylformamid und 5,5 ml Wasser versetzt. Nach Zugabe von 0,82 g (12,66 mmol) Natriumazid wird der Ansatz über Nacht gerührt. Die Reaktionsmischung wird auf Wasser gegeben und dreimal mit Methyl-tert. Butylether extrahiert. Die vereinigten organischen Extrakte werden mit Wasser und mit Sole gewaschen. Nach dem Trocknen über Natriumsulfat wird filtriert und das Lösungsmittel abrotiert. Chromatographie am Flashmaster ergibt 2,06 g (95,8%) des gewünschten Azids.
¹H-NMR (300 MHz, CDCl₃): δ = 4.00 (3H), 4.90 (2H), 7.73 (1 H), 7.87 (1 H).

### 4-Amino-6-fluor-2,3-dihydroisoindol-1-on

1,86 g (7,32 mmol) 2-(Azidomethyl)-5-fluor-3-nitrobenzoesäuremethylester werden in 46 ml Ethanol und 3,4 ml Eisessig gegeben und mit 256,6 mg Pd/C versetzt. Nach dem Rühren über Nacht bei Raumtemperatur unter einer Wasserstoffatmosphäre wird der Katalysator über ein Glasfaserfilter abgesaugt und das Filtrat bis zur Trockene eingeengt. Der Rückstand, 1,18 mg (97,5%)der gewünschten Verbindung, wird roh weiter eingesetzt.
¹H-NMR (300 MHz, DMSO-d₆): δ = 4.10 (2H), 5.75 (2H), 6.46-6.57 (2H), 8.50 (1 H).

### 4-(4-Chlor-2-methoxyphenyl)-2-hydroxy-4-methyl-2-(trifluormethyl)pentan-1-ol

Eine Lösung von 3 g 2-Hydroxy-4-methylen-2-(trifluormethyl)valeriansäureethylester in 22 ml 3-Chloranisol werden bei RT portionsweise mit Aluminiumtrichlorid versetzt. Nach 48stündigem Rühren bei Raumtemperatur, wird der Ansatz mit 2 N Salzsäure und Hexan versetzt und eine weitere Stunde gerührt. Nach Waschen mit 2 N Salzsäure und Wasser wird überschüssiges 3-Chloranisol im Vakuum abdestilliert. Der verbleibende Rückstand wird durch Chromatographie an Kieselgel (Laufmittel Hexan/Essigester) gereinigt. Man erhält 2.85 g eines Gemisches von 4-(4-Chlor-2-methoxyphenyl)-2-hydroxy-4-methyl-2-(trifluormethyl)valeriansäureethylester und der regioisomeren Verbindung 4-(2-Chlor-4-methoxyphenyl)-2-hydroxy-4-methy-2-(trifluormethyl)valeriansäureethylester als gelbes Öl. Dieses Substanzgemisch wird in 90 ml Ether bei 0°C mit 445 mg Lithiumaluminiumhydrid versetzt und 12 h gerührt. Der Ansatz wird auf gesättigte Natriumhydrogencarbonat-Lösung gegeben und durch Kieselgur filtriert. Die Phasen werden getrennt und die wässrige Phase mit Essigester extrahiert. Es wird mit Wasser und Sole gewaschen, mit Natriumsulfat getrocknet und im Vakuum eingeengt. Nach Chromatographie an Kieselgel (Laufmittel Hexan/Essigester) erhält man als 1. Fraktion 1.87 g der gewünschten Verbindung 4-(4-Chlor-2-methoxyphenyl)-2-hydroxy-4-methyl-2-(trifluormethyl)pentan-1-ol und als 2. Fraktion 160 mg der regioisomeren Verbindung 4-(2-Chlor-4-methoxyphenyl)-2-hydroxy-4-methyl-2-(trifluormethyl)pentan-1-ol als farblose Öle.
1. Fraktion: ¹H-NMR (300 MHz, CDCl₃), δ = 1.41 (s, 3H), 1.51 (s, 3H), 2.24 (d, 1 H), 2.51 (d, 1 H), 2.84 (bs, 1 H), 3.36 (d, 1 H), 3.48 (d, 1 H), 3.85 (s, 3H), 6.88 (d, 1 H), 6.92 (dd, 1 H), 7.24 (d, 1 H)
2. Fraktion: ¹H-NMR (300 MHz, CDCl₃), δ =1.52 (s, 3H), 1.62 (s, 3H), 2.18 (d, 1 H), 2.76 (d, 1 H), 2.93 (bs, 1 H), 3.33 (d, 1 H), 3.55 (d, 1 H), 3.80 (s, 3H), 6.78 (dd, 1 H), 6.90 (d, 1 H), 7.38 (d, 1 H)

### 4-(4-Chlor-2-methoxyphenyl)-2-hydroxy-4-methyl-2-(trifluormethyl)pentanal

In einen ausgeheizten Kolben werden 854,6 mg (6,733 mmol) Oxalylchlorid in 14,5 ml Dichlormethan vorgelegt vorgelegt. Bei -70°C werden 1,05 ml DMSO, gelöst in 3 ml Dichlormethan, zugetropft und der Ansatz fünf Minuten nachgerührt. Anschließend werden 2 g (6,12 mmol) 4-(4-Chlor-2-methoxyphenyl)-2-hydroxy-4-Methyl-2-trifluormethyl-pentan-1-ol, gelöst in sechs Millilitern Dichlormethan zugetropft. Nach 20minütigem Rühren wird der Ansatz vorsichtig mit 4,24 ml (30,61 mmol) Triethylamin versetzt, und zwar in einem Temperaturbereich zwischen -70 und -60°C. Nach fünfminütigem Rühren bei -70°C lässt man die Reaktionsmischung langsam auf Raumtemperatur kommen lassen. Es werden
25 ml Wasser zugegeben und der Ansatz eine weiter Stunde bei Raumtemperatur gerührt. Nach Phasentrennung wird die wässrige Phase einmal mit 100 ml Dichlormethan geschüttelt. Die vereinigten organischen Extrakte werden mit 1 %iger Schwefelsäure, 5%iger Natriumhydrogencarbonatlösung und Sole gewaschen. Nach dem üblichen Procedere werden 1,92 g (96,9%) des gewünschten Aldehyds erhalten, der roh in die nächste Stufe eingesetzt wird.
¹H-NMR (300 MHz, CDCl₃): δ = 1.37 (3H), 1.45 (3H), 2.22 (1 H), 3.35 (1 H), 3.59 (1 H), 3.90 (3H), 6.80-6.92 (2H), 7.04 (1 H), 9.02 (1 H).

### 4-{[4-(4-Chlor-2-methoxyphenyl)-2-hydroxy-4-methyl-2-trifluormethyl)pentyliden]amino}6-fluor-2,3-dihydroisoindol-1-on

250 mg (0,769 mmol) rac-4-(4-Chlor-2-methoxyphenyl)-2-hydroxy-4-methyl-2-(trifluormethyl)-pentanal werden mit 127,9 mg (0,769 mmol) 4-Amino-6-fluor-2,3-dihydroisoindol-1-on in 1,12 ml Eisessig vier Tage bei Raumtemperatur gerührt. Die Mischung wird dreimal mit Toluol versetzt und am Rotationsverdampfer bis zur Trockene eingeengt. Der Rückstand wird an Kieselgel (Laufmittel Ethylacetat/Hexan) chromatographiert. Isoliert werden 236,7 mg (65%) der gewünschten Verbindung.
¹H-NMR (300 MHz, CDCl₃): δ =1.38 (3H), 1.53 (3H), 2.21 (1 H), 3.40 (1 H), 3.90 (3H), 4.30 (2H), 4.54 (1 H), 6.22 (1 H), 6.70 (1 H), 6.78 (1 H), 6.89 (1 H), 7.04 (1 H), 7.45 (1 H), 7.49 (1 H).

### 4-{[1-(4-Chlor-2-methoxyphenyl)-2-hydroxy-4-methyl-2-(trifluormethyl)-pent-4-en-1-yl]amino}-6-fluor-2,3-dihydroisoindol-1-on)

145 mg (0,307 mmol) 4-([4-(4-Chlor-2-methoxyphenyl)-2-hydroxy-4-methyl-2-trifluormethyl)pentyliden]amino}6-fluor-2,3-dihydroisoindol-1-on werden bei -30°C mit 6,1 ml einer 1 M Lösung von BBr₃ in Dichlormethan versetzt und anderthalb Stunden bei -20°C gerührt. Das Reaktionsgemisch wird bei 20°C tropfenweise mit gesättigter Natriumhydrogencarbonatlösung versetzt bis pH 8. Nach dem Versetzen mit Ethylacetat wird das Kältebad entfernt und 15 Minuten gerührt. Nach dem Extrahieren mit Ethylacetat (zweimal) werden die vereinigten organischen Extrakte mit Wasser und Sole gewaschen und getrocknet (Natriumsulfat). Nach dem Abfiltrieren des Trockenmittels und Abrotieren des Lösungsmittels wird der Rückstand an Kieselgel (Laufmittel Methanol/Dichlormethan) chromatographiert. Isoliert werden 25 mg (17,2%) der gewünschten Verbindung.
¹H-NMR (300 MHz, CD₃OD): δ = 1.78 (3H), 2.22 (1 H), 2.48 (1 H), 4.02 (3H), 4.22-4.43 (2H), 4.67 (1 H), 4.82 (1 H), 5.25 (1 H), 6.31 (1 H), 6.71 (1 H), 6.99 (1 H), 7.10 (1 H), 7.52 (1 H).

### Beispiel 2

### 4-{[1-(5-Chlor-2-hydroxyphenyl)-2-hydroxy-4-methyl-2-(trifluormethyl)-pent-4-en-1-yl]amino}-6-fluor-2,3-dihydroisoindol-1-on

### 4-{[4-(5-Chlor-2-methoxyphenyl)-2-hydroxy-4-methyl-2-trifluormethyl)pentyliden]amino}6-fluor-2,3-dihydroisoindol-1-on

250 mg (0,769 mmol) rac-4-(5-Chlor-2-methoxyphenyl)-2-hydroxy-4-methyl-2-(trifluormethyl)-pentanal werden mit 127,9 mg (0,769 mmol) 4-Amino-6-fluor-2,3-dihydroisoindol-1-on in
1,12 ml Eisessig vier Tage bei Raumtemperatur gerührt. Die Mischung wird dreimal mit Toluol versetzt und am Rotationsverdampfer bis zur Trockene eingeengt. Der Rückstand wird an Kieselgel (Laufmittel Ethylacetat/Hexan) chromatographiert. Isoliert werden 244,8 mg (67,2%) der gewünschten Verbindung.
¹H-NMR (300 MHz, CDCl₃): δ = 1.38 (3H), 1.53 (3H), 2.20 (1 H), 3.49 (1 H), 3.89 (3H), 4.38 (2H), 4.60 (1 H), 6.26 (1 H), 6.68-6.80 (2H), 6.95 (1 H), 7.02 (1 H), 7.39-7.48 (2H).

### 4-{[1-(5-Chlor-2-hydroxyphenyl)-2-hydroxy-4-methyl-2-(trifluormethyl)-pent-4-en-1-yl]amino}-6-fluor-2,3-dihydroisoindol-1-on

140 mg (0,296 mmol) 4-{[4-(5-Chlor-2-methoxyphenyl)-2-hydroxy-4-methyl-2-trifluormethyl)pentyliden]amino}6-fluor-2,3-dihydroisoindol-1-on werden mit 2,96 ml einer
1 M Lösung von BBr₃ in Dichlormethan versetzt und zwanzig Stunden bei Raumtemperatur gerührt. Nach dem im Beispiel 1 beschriebenen Aufarbeiten wird der Rückstand mit Hilfe des Flashmasters chromatographiert (Laufmittel Methanol/Dichlormethan). Isoliert werden 71 mg (52,3%) der gewünschten Verbindung.
¹H-NMR (300 MHz, CD₃OD): δ = 1.78 (3H), 2.25 (1 H), 2.59 (1 H), 4.42-4.45 (2H), 4.69 (1 H), 4.82 (1 H), 5.22 (1 H), 6.47 (1 H), 6.73 (1 H), 6.87 (1 H), 7.13 (1 H), 7.48 (1 H).

### Beispiel 3

### 5-{[1-(5-Brom-2-hydroxyphenyl)-2-hydroxy-4-methyl-2-(trifluormethyl)-pent-4-en-1-yl]amino}-isochinolin-1 (2H)-on

### 5-Amino-isochinolin-1 (2H)-on

### 5-Nitroisocoumarin

16,4 g (84,03 mmol) des unter Beispiel 1 beschriebenen 2-Methyl-3-nitrobenzoesäuremethylesters werden mit 26,8 g (225,1 mmol) N, N-Dimethylformamiddimethylacetal in 85 ml Dimethylformamid 12 Stunden bei 130°C gerührt. Das Lösungsmittel wird am Rotationsverdampfer abgezogen, der Rückstand in Methyl-, tert.-butylether aufgenommen und dreimal mit Wasser gewaschen. Nach dem Waschen mit gesättigter NaCl-Lösung wird die organische Phase getrocknet. Nach Abfiltrieren des Trockenmittels und Abrotieren des Lösungsmittels wird der verbleibende Rückstand an Kieselgel (Laufmittel Ethylacetat/Hexan) chromatographiert. Isoliert werden 8,73 g (54,4%) der gewünschten Verbindung.
¹H-NMR (300 MHz, CDCl₃): δ = 7.39 (1 H), 7.45 (1 H), 7.68 (1 H), 8.49 (1 H), 8.65 (1 H).

### 5-Nitroisochinolin-1 (2H)-on

2,51 g (13,13 mmol) 5- Nitroisocoumarin werden in 100 ml Ethanol gegeben. Unter Druck wird im Autoklaven Ammoniak eingedrückt. Das Produkt fällt aus und wird abgesaugt. Isoliert werden 1,98 g (79,7%) der gewünschten Verbindung. ¹H-NMR (300 MHz, DMSO-d₆): δ = 6.97 (1 H), 7.45 (1 H), 7.65 (1 H), 8.43 (1 H), 8.57 (1 H),
11.5 (1 H).

### 5-Aminoisochinolin-1 (2H)-on

268,3 mg (1,51 mmol) 5-Nitroisochinolin-1 (2H)-on werden mit 376,5 mg Ammoniumchlorid und 2,6 ml Wasser in 14 ml Ethanol und 5,4 ml Tetrahydrofuran gegeben. Nach portionsweiser Zugabe von 1,23 g Zinkpulver (Erwärmung auf 30 bis 35°C) werden zwei Stunden gerührt. Das Reaktionsgemisch wird über Glasfaserfilter abgesaugt und mit Ethylacetat nachgewaschen. Nach dem Waschen des Filtrats mit Wasser und gesättigter Natriumchloridlösung wird die organische Phase wie üblich getrocknet. Abfiltrieren des Trockenmittels und Abrotieren des Lösungsmittels ergeben 196,5 mg (88,1%) des gewünschten Amins.
¹H-NMR (300 MHz, DMSO-d₆): δ = 5.6 (2H), 6.68 (1H), 6.87.45 (1H), 7.00 (1H), 7.17 (1 H), 7.39 (1 H), 11.7 (1 H).

### 5-{[4-(5-Brom-2-methoxyphenyl)-2-hydroxy-4-methyl-2-(trifluormethyl)pentyliden]amino}-isochinolin-1(2H)-on

400 mg (1,297 mmol) rac-4-(5-Brom-2-methoxyphenyl)-2-hydroxy-4-methyl-2-trifluormethyl)-pentanal werden in 1,57 ml Eisessig mit 173,5 mg (1,083 mmol) 5-Aminoisochinolin-1 (2H)-on sechs Tage bei Raumtemperatur gerührt. Die Mischung wird dreimal mit Toluol versetzt und am Rotationsverdampfer bis zur Trockene eingeengt. Der Rückstand wird an Kieselgel (Laufmittel Ethylacetat/Hexan) chromatographiert. Isoliert werden 195 mg (35,2%) der gewünschten Verbindung.
¹H-NMR (300 MHz, CDCl₃): δ = 1.38 (3H), 1.53 (3H); 2.29 (1 H), 3.50 (1 H), 3.83 (1 H), 4.83 (1 H); 6.50-6.62 (2H), 6.75 (1 H), 7.04 (1 H), 7.16 (1 H), 7.19-7.30 (1 H), 7.31-7.43 (2H), 8.32 (1H), 10.83 (1 H).

### 5-([1-(5-Brom-2-hydroxyphenyl)-2-hydroxy-4-methyl-2-(trifluormethyl)-pent-4-en-1-yl]amino}-isochinolin-1 (2H)-on

195 mg (0,381 mmol) 5-([4-(5-Brom-2-methoxyphenyl)-2-hydroxy-4-methyl-2-(trifluormethyl)pentyliden]amino)-isochinolin-1 (2H)-on werden mit 3,8 ml einer 1 M Lösung von BBr₃ in Dichlormethan versetzt und 90 Minuten bei Raumtemperatur gerührt. Die Reaktionsmischung wird auf Eis gegeben, mit Natriumhydrogencarbonatlösung auf pH 8 gebracht und, wie im Beispiel 1 beschrieben, weiter aufgearbeitet. Der Rückstand wird gemeinsam mit dem Rückstand aus einem weiteren Ansatz, in den 145 mg (0,283 mmol) rac-5-{(E/Z)-[4-(5-Brom-2-methoxyphenyl)-2-hydroxy-4-methyl-2-(trifluormethyl)pentyliden]amino}-isochinolin-1(2H)-on eingesetzt worden ist, mit Hilfe des Flashmasters chromatographiert (Aminphase; Laufmittel Dichlormethan/Methanol) chromatographiert. Isoliert werden 45,1 mg (13,64%) der gewünschten Verbindung.
¹H-NMR (300 MHz, CD₃OD): δ = 1.80 (3H), 2.39 (1 H), 2.60 (1 H), 4.68 (1 H), 4.82 (1 H), 5.22 (1 H), 6.63-6.89 (3H), 7.12-7.30 (3H), 7.50 (1 H), 7.60 (1 H).

### Beispiel 4

### (rac.) 4-(fi-(5-Fluor-2-hvdroxypheny)-2-hydroxy-4-methyl-2-Lrifluormethyl)-pent-4-en-1-yl]amino)-2,3-dihydroisoindol-1-on und

### (rac.) 4-{[1-(5-Fluor-2-hydroxyphenyl)-2-hydroxy-4-methyl-2-(trifluormethyl)-pent-3-en-1-yl]amino)-2,3-dihydroisoindol-1-on

### 4-(5-Fluor-2-methoxyphenyl)-2-hydroxy-4-methyl-2-trifluormethyl)-pentanal

6,55g (21,11 mmol) rac-4-(5-Fluor-2-methoxyphenyl)-2-hydroxy-4-methyl-2-trifluormethyl)-pentan-1-ol (Name) (WO 00/32584) werden in 224 ml Dichlormethan gelöst und bei Raumtemperatur mit 74 ml trockenem Dimethysulfoxid und 10,68 g (105,55 mmol) Triethylamin versetzt. Bei 15 bis 18°C werden innerhalb von 40 Minuten 10,08 g
(63,33 mmol) des SO₃/Pyridinkomplexes portionsweise zugegeben. Nach dem Rühren über Nacht bei Raumtemperatur werden 84 ml gesättigte Ammoniumchloridlösung zugegeben. Es tritt eine leichte Erwärmung auf. Nach 15minütigem Rühren bei Raumtemperatur wird zweimal mit je 300 ml Diethylether extrahiert. Die organischen Phasen werden mit Wasser und Sole gewaschen und getrocknet (Natriumsulfat). Nach dem Abfiltrieren des Lösungsmittels und Abrotieren des Lösungsmittels wird der verbleibende Rückstand an Kieselgel (Laufmittel Ethylacetat/Hexan) chromatographiert. Isoliert werden 5,85 g (90%) der gewünschten Verbindung.
¹H-NMR (300 MHz, CDCl₃): δ = 1.40 (3H), 1.46 (3H), 2.22 (1 H), 3.38 (1 H), 3.59 (1 H), 3.86 (1 H), 6.70-6.80 (1 H), 6.82-6.97 (2H), 9.05 (1 H).

### 4-{[4-(5-Fluor-2-methoxyphenyl)-2-hydroxy-4-methyl-2-(trifluormethyl)pentyliden]amino}2,3-dihydroisoindol-1-on

400 mg (1,297 mmol) 4-(5-Fluor-2-methoxyphenyl)-2-hydroxy-4-methyl-2-trifluormethyl)-pentanal werden mit 192,1 mg (1,297 mmol) 4-Amino-2,3-dihydroisoindol-1-on in 1,89 ml Eisessig vier Tage bei Raumtemperatur gerührt. Die Mischung wird dreimal mit Toluol versetzt und am Rotationsverdampfer bis zur Trockene eingeengt. Der Rückstand wird an Kieselgel (Laufmittel Ethylacetat/Hexan) chromatographiert. Isoliert werden 429,7 mg (75,5%) der gewünschten Verbindung.
¹H-NMR (300 MHz, CDCl₃): δ = 1.37 (3H), 1.52 (3H), 2.22 (1 H), 3. 42 (1 H), 3.84 (3H), 4.37 (2H), 4.68 (1 H), 6.53-6.68 (3H), 6.72-6.95 (2H), 7.37 (1 H), 7.49 (1 H), 7.75 (1 H).

### 4-{[1-(5-Fluor-2-hydroxyphenyl)-2-hydroxy-4-methyl-2-(trifluormethyl)-pent-4-en-1-yl]amino}-2,3-dihydroisoindol- 1-on

420 mg (0,958 mmol) der im vorigen Absatz beschriebenen Verbindung 4-{[4-(5-Fluor-2-methoxyphenyl)-2-hydroxy-4-methyl-2-(trifluormethyl)pentyliden]amino}2,3-dihydroisoindol-1-on werden mit 9,6 ml einer 1 M Lösung von Bortribromid in Dichlormethan versetzt und eindreiviertel Stunde bei Raumtemperatur gerührt. Das Reaktionsgemisch wird bei -30°C tropfenweise mit gesättigter Natriumhydrogencarbonat bis pH 8 versetzt. Nach dem Verdünnen mit Ethylacetat wird das Kältebad entfernt und 15 Minuten kräftig gerührt. Nach zweimaligem Extrahieren mit Ethylacetat werden die organischen Phasen mit Wasser und gesättigter Natriumchloridlösung gewaschen. Nach dem Trocknen (Natriumsulfat) und Abrotieren des Lösungsmittels wird der Rückstand am Flashmaster (Kieselgel, Flash NH₂) chromatographiert (Laufmittel Dichlormethan/Methanol). Isoliert werden 37,8 mg (9,3%) der gewünschten Verbindung.
¹H-NMR (300 MHz, CD₃OD): δ = 1.82 (3H), 2.38 (1 H), 2.55 (1 H), 4.28-4.50 (2H), 4.69 (1 H), 4.81 (1 H), 5.20 (1 H), 6.70-6.87 (3H), 7.06-7.17 (2H), 7.22 (1 H).
Außerdem werden 12,8 mg der regioisomeren Verbindung 4-{[1-(5-Fluor-2-hydroxyphenyl)-2-hydroxy-4-methyl-2-(trifluormethyl)-pent-3-en-1-yl]amino}-2,3-dihydroisoindol-1-on isoliert.
Smp.: 195-197° C

### Beispiel 5

### (rac.) 4-{[1-(5-Fluor-2-hydroxyphenyl)-2-hydroxy-4-methyl-2-(trifluormethyl)-pent-4-en-1-yl]amino}-6-fluor-2,3-dihydroisoindol-1-on

### 4-{(4-(5-Fluor-2-methoxyphenyl)-2-hydroxy-4-methyl-2-(trifluormethyl)pentyliden]amino)6-fluor-2,3-dihydroisoindol-1-on

380 mg (0.832 mmol) des in Beispiel 4 beschriebenen rac-4-(5-Fluor-2-methoxyphenyl)-2-hydroxy-4-methyl-2-trifluormethyl)-pentanals werden in 1,89 ml Eisessig mit 215,4 mg (1,297 mmol) des in Beispiel eins beschriebenen 4-Amino-6-fluor-2,3-dihydroisoindol-1-on umgesetzt und vier Tage bei Raumtemperatur gerührt. Da laut DC noch Ausgangsmaterial vorhanden ist, wird die Reaktionsmischung mit Toluol versetzt und 20 Stunden am Wasserabscheider gekocht. Das Toluol wird am Rotationsverdampfer abgezogen und der Rückstand an Kieselgel (Laufmittel Ethylacetat/Hexan) chromatographiert. Isoliert werden 383,4 mg (64,7%) des gewünschten Imins.
¹H-NMR (300 MHz, CDCl₃): δ = 1.37 (3H), 1.53 (3H), 2.20 (1 H), 3.47 (1 H), 3.88 (3H), 4.32 (2H), 4.57 (1 H), 6.22 (1 H), 6.63-6.88 (4H), 7,42 (1 H), 7.48 (1 H).

### 4-{[1-(5-Fluor-2-hydroxyphenyl)-2-hydroxy-4-methyl-2-(trifluormethyl)-pent-4-en-1-yl]amino}-6-fluor-2,3-dihydroisoindol- 1-on

380 mg (0,832 mmol) 4-{[4-(5-Fluor-2-methoxyphenyl)-2-hydroxy-4-methyl-2-(trifluormethyl)pentyliden]amino}6-fluor-2,3-dihydroisoindol-1-on werden bei Raumtemperatur mit 8,3 ml einer 1 M Lösung von BBr₃ in Dichlormethan versetzt und eine Stunde bei Eisbadtemperatur gerührt. Die Aufarbeitung des Ansatzes erfolgt wie
im Beispiel vier beschrieben. Nach Chromatographie des Rohprodukts am Flashmaster (Aminphase; Laufmittel Methanol/Dichlormethan) werden 22,2 mg (6,03%) der gewünschten Verbindung erhalten.
¹H-NMR (300 MHz, CD₃OD): δ =1.80 (3H), 2.30 (1 H), 2.55 (1 H), 4.22-4.48 (2H), 4.68 (1 H), 4.81 (1 H), 5.17 (1 H), 6.49 (1 H), 6.72 (1 H), 6.75-6.90 (2H), 7.17 (1 H).

### Beispiel 6

### 2-{4-Chlor-alpha-[(2-methylchinazolin-5-yl)amino]-2-methoxybenzyl}-1,1,1-trifluor-4-methylpent-4-en-2-ol und

### 2-{4-Chlor-alpha-[(2-methylchinazolin-5-yl)aminol-2-methoxy_{.}benzyll-1,1,1-trifluor-4-methylpent-3-en-2-ol

### 5-Amino-2-methychinazolin

12,7g (mmol) 2-Methyl-5-nitro-3H-chinazolin-4-on (M.T. Bogert, V.J. Chambers *J*. *Org Chem.* **1905**, 649-658) und 37,5 g Phosphorpentachlorid werden in 75 ml Phosphorylchlorid über 20 Stunden unter Rückfluss erhitzt. Nach dem Abkühlen gießt man in ges. NaHCO₃ Lösung und extrahiert mit Ethylacetat. Die organische Phase wird getrocknet und das Lösungsmittel entfernt. Man erhält 14 g 4-Chlor-2-methyl-5-nitrochinazolin, von denen 4.5 g (20.2 mmol) in 225 ml Ethylacetat und 22.5 ml Triethylamin gelöst werden. Man gibt 2 g Palladium auf Kohle zu und rührt bei Eiskühlung vier Stunden unter einer Wasserstoffatmosphäre bei Normaldruck. Die Lösung wird mittels Filtration über Celite vom Katalysator befreit, wobei mit 200 ml Ethanol nachgewaschen wird, und eingedampft. Nach Chromatographie an Kieselgel mit Essigester-Ethanol (0-10%) werden 530 mg des Produkts erhalten.
¹H-NMR (300 MHz, CDCl₃); δ = 2.87 (s, 3H), 4.52 (br., 2H), 6.77 (d, 1 H), 7.33 (d, 1 H), 7.65 (t, 1 H), 9.40 (s, 1 H).

### 1,1,1,-Trifluor-4-(4-chlor-2-methoxyphenyl)-2-[(E/Z)-(2-methyl-chinazol-5-yl)iminomethyl]-4-methyl-pentan-2-ol

325 mg (1.00 mmol) rac-4-(4-Chlor-2-methoxyphenyl)-2-hydroxy-4-methyl-2-(trifluormethyl)-pentanal werden mit 200 mg (1.25 mmol) 5-Amino-2-methylchinazolin in 5 ml Toluol gelöst und bei RT 0.74 ml (2.50 mmol) Titantetraisopropylat zugesetzt. Die Mischung wird 3 h bei 105°C gerührt. Nach dem Abkühlen wird die Mischung auf Wasser gegeben, einige min gerührt, über Kieselgur abgesaugt, mit Ethylacetat nachgewaschen und die Phasen getrennt. Die wässrige Phase wird mehrfach mit Ethylacetat extrahiert und die vereinigten organischen Phasen mit ges. NaCl-Lösung gewaschen und mit Natriumsulfat getrocknet. Die Lösungsmittel werden am Rotationsverdampfer entfernt und das Rohprodukt an Kieselgel chromatographiert (Eluent Hexan/Ethylacetat 4:1). Isoliert werden 130 mg (28%) der gewünschten Verbindung.

### (rac.) 2-{4-Chlor-alpha-[(2-methylchinazolin-5-yl)amino]-2-methoxybenzyl}-1,1,1-trifluor-4-methylpent-4-en-2-ol:

130 mg (0.28 mmol) 1,1,1,-Trifluor-4-(4-chlor-2-methoxyphenyl)-2-[(2-methyl-chinazol-5-yl)iminomethyl]-4-methyl-pentan-2-ol werden in 5 ml Dichlormethan vorgelegt und bei -70 °C 5.6 ml einer 1 M Lösung von Bortribromid in Dichlormethan zugetropft. Man lässt innerhalb von 1.5 h auf -10 °C kommen, gibt dann 5 ml einer ges. NaHCO₃₋Lösung zu, trennt die Phasen, extrahiert die wässrige Phase mit Dichlormethan, wäscht die vereinigten organischen Phasen mit ges. NaHCO₃-Lösung und ges. NaCl-Lösung und trocknet mit Natriumsulfat. Nach Entfernen des Lösungsmittels am Rotationsverdampfer wird das Rohprodukt an Kieselgel (Eluent: Dichlormethan/Methanol 98:2) chromatographiert. Man erhält 80 mg (62%) des gewünschten Produkts als gelben Feststoff.
Schmp.: 193 °C.
Zudem erhält man als geringfügig polareres Nebenprodukt 7 mg der doppelbindungsisomeren Verbindung 2-{4-Chlor-alpha-[(2-methylchinazolin-5-yl)amino]-2-methoxybenzyl}-1,1,1 -trifluor-4-methylpent-3-en-2-ol.
¹H-NMR (300 MHz, CD₃OD): δ = 1.35 (s, 3H), 1.61 (s, 3H), 2.80 (s, 3H), 3.98 (s, 3H), 5.24 (s, 1 H), 5.35 (s, 1 H), 6.35 (d, J = 8 Hz, 1 H), 6.87 (dd, J = 2/8 Hz, 1 H), 6.97 (d, J = 2 Hz, 1 H), 7.09 (d, J = 8 Hz, 1 H), 7.49 (d, J = 8 Hz, 1 H), 7.60 (dd, J = 8/8 Hz, 1 H), 9.58 (s, 1 H).

### Beispiel 7

### (rac.) 2-{4-Chlor-alpha-[(8-fluor-2-methylchinazolin-5-yl)amino]-2-methoxybenzyl}-1,1,1-trifluor-4-methylpent-4-en-2-ol und

### (rac.) 2-{4-Chlor-alpha-[(8-fluor-2-methylchinazolin-5-yl)amino]-2-methoxybenzyl}-1,1,1-trifluor-4-methylpent-3-en-2-ol

### 5-Amino-8-fluor-2-methylchinazolin

Zu einer Lösung von 3.35 g (20.25 mmol) Chloralhydrat und 21.27 g (149.7 mmol) Natriumsulfat in 72 ml Wasser wird eine 50°C warme Lösung von 2.4 g (18.6 mmol) 2,5-Difluoranilin in 11 ml Wasser und 1.6 ml konz. Salzsäure (37%) gegeben, die bei dieser Temperatur vorher 1 h gerührt wurde. Man rührt weitere 30 min bei RT und erhitzt nach der Zugabe von 4.09 g (58.9 mmol) Hydroxylammoniumchlorid in 19 ml Wasser über 45 min auf 125°C und hält diese Temperatur für 5 min. Nach dem Abkühlen und einer weiteren Stunde wird der ausgefallene hellbraune Niederschlag abfiltriert, mit Wasser gewaschen und getrocknet. Man erhält 3.0g (15.0 mmol) des Hydroxylimins als Zwischenprodukt, die portionsweise in 15 ml konz. Schwefelsäure bei 60°C gelöst werden. Nach vollständiger Zugabe erhitzt man 2 Stunden auf 80°C und 4h auf 90°C. Man läßt abkühlen und gießt die Lösung auf 100g Eis. Man extrahiert mit Ethylacetat, wäscht die organische Phase mit Wasser, trocknet über Natriumsulfat und engt ein. Nach Chromatographie an Kieselgel mit Hexan-Essigester (0-45%) werden 1,2 g (7.1 mmol) des 4,7-Difluorisatins erhalten. Zum Isatin in 30 ml einer 1 molaren Natronlauge werden über 10 min 1.8 ml einer 30%igen Wasserstoffperoxid Lösung getropft. Nach 2 Stunden Rühren bei RT wird auf 0°C gekühlt und es werden 5 ml einer 4 molaren Salzsäure zugegeben und mit 50 ml Wasser verdünnt. Man extrahiert mit Ethylacetat, trocknet über Natriumsulfat, engt ein und erhält so quantitativ 1.27 g der 3,6-Difluoranthranilsäure, die ohne weitere Aufreinigung umgesetzt wird.

Die 3,6-Difluoranthranilsäure wird in 8 ml Essigsäureanhydrid 45 min lang auf 100°C erhitzt. Nach dem Abkühlen wird die entstandene Essigsäure und überschüssiges Essigsäureanhydrid azeotrop mit Toluol im Vakuum entfernt. Der Rückstand wird unter Eiskühlung mit 40 ml einer 25%igen Ammoniaklösung versetzt und 72 Stunden gerührt. Man verdünnt mit Wasser und säuert mit Essigsäure an. Man extrahiert mit Ethylacetat, wäscht die organische Phase mit Wasser, trocknet über Natriumsulfat und engt ein. Die so erhaltenen 1,03 g (5.25 mmol) 5,8-Difluor-2-methyl-3H-chinazolin-4-on und 6 g Phosphorpentachlorid werden in 20 ml Phosphorylchlorid über 12 h auf 125°C erhitzt. Nach dem Abkühlen gießt man in ges. NaHCO₃ Lösung und extrahiert mit Ethylacetat. Die organische Phase wird getrocknet und das Lösungsmittel entfernt. Man erhält quantitativ 1.7g 4-Chlor-5,8-difluor-2-methylchinazolin, die in 60 ml Ethylacetat und 5 ml Triethylamin gelöst werden. Man gibt 600 mg Palladium auf Kohle zu und schüttelt 2 h (480 ml Wasserstoffaufnahme) unter einer Wasserstoffatmosphäre bei Normaldruck. Die Lösung wird mittels Filtration über Celite vom Katalysator befreit, wobei mit 100 ml Ethanol nachgewaschen wurde, und eingedampft. Nach Chromatographie an Kieselgel mit Hexan-Essigester-Ethanol (0-40%) werden 550 mg 5,8-Difluor-2-methylchinazolin erhalten. Zu 240 mg (1.3 mmol) 5,8-Difluor-2-methylchinazolin, 300 mg (1.13 mmol) 18-Krone-6 in 10 ml DMF werden 890 mg (13.7 mmol) Natriumazid gegeben und man erhitzt die Mischung über 8 h auf 125°C. Das Lösungsmittel wird im Vakuum entfernt und man chromatographiert an Kieselgel mit Ethylacetat und erhält 52 mg Produkt.
¹H-NMR (300 MHz, CDCl₃); δ = 2.92 (s, 3H), 4.31 (br., 2H), 6.67 (dd, 1 H), 7.38 (dd, 1 H), 9.37 (s, 1H).

### (rac) 1,1,1,-Trifluor-4-(4-chlor-2-methoxyphenyl)-2-[(8-fluor-2-methyl-chinazol-5-yl)iminomethyl]-4-methyl-pentan-2-ol

2.40 g (7.39 mmol) *rac*-4-(4-Chlor-2-methoxyphenyl)-2-hydroxy-4-methyl-2-(trifluormethyl)-pentanal werden mit 1.30 g (7.34 mmol) 5-Amino-8-fluor-2-methylchinazolin in 30 ml Toluol gelöst und bei RT 3.10 ml (14.7 mmol) Titantetraethylat zugesetzt. Die Mischung wird 3 h bei 105 °C gerührt. Nach dem Abkühlen wird die Mischung auf Wasser gegeben, einige min gerührt, über Kieselgur abgesaugt, mit Ethylacetat und Wasser nachgewaschen und die Phasen getrennt. Die wässrige Phase wird mehrfach mit Ethylacetat extrahiert und die vereinigten organischen Phasen mit ges. NaCl-Lösung gewaschen und mit Natriumsulfat getrocknet. Die Lösungsmittel werden am Rotationsverdampfer entfernt und das Rohprodunkt an Kieselgel chromatographiert (Eluent Hexan/Ethylacetat 4:1). Isoliert werden 3.20 g (90%) der gewünschten Verbindung.

### (rac.) 2-{4-Chlor-alpha-[(8-fluor-2-methylchinazolin-5-yl)amino]-2-methoxybenzyl]-1,1,1-trifluor-4-methylpent-4-en-2-ol

3.1 g (6.4 mmol) (rac) 1,1,1,-Trifluor-4-(4-chlor-2-methoxyphenyl)-2-[(8-fluor-2-methylchinazol-5-yl)iminomethyl]-4-methyl-pentan-2-ol werden in 50 ml Dichlormethan vorgelegt und bei -75°C 128 ml einer 1 M Lösung von Bortribromid in Dichlormethan zugetropft. Man lässt innerhalb von 2.5 h auf -20°C kommen. Dann gibt man die Reaktionsmischung auf 300 ml einer ges. NaHCO₃-Lösung, rührt 15 min nach, trennt die Phasen, extrahiert die wässrige Phase mit Dichlormethan, wäscht die vereinigten organischen Phasen mit ges. NaHCO₃-Lösung und ges. NaCl-Lösung und trocknet mit Natriumsulfat. Nach Entfernen des Lösungsmittels am Rotationsverdampfer wird das Rohprodukt an Kieselgel (Eluent: Hexan/Ethylacetat 3:1 bis 1:1) chromatographiert. Anschließend wird nochmals mittels HPLC gereinigt Man erhält 650 mg (26%) des gewünschten Produkts als gelben Feststoff.
Schmp.: 110°C.
Zudem kann als geringfügig polareres Nebenprodukt die doppelbindungsisomere Verbindung *(rac.)* 2-{4-Chlor-alpha-[(8-fluor-2-methylchinazolin-5-yl)amino]-2-methoxybenzyl}-1,1,1-trifluor-4-methylpent-3-en-2-ol isoliert werden.
Schmp.: 118°C
Das Hauptprodukt wird in seine Enantiomeren getrennt: 1. Chiralpak AD 20µ, Hex/EtOH 95/5, 2. Luna C18, CH₃CN/H₂O 50/50.
MS (ESI): 484/486 (M+1), 516/518 (M+1+MeOH);
¹H-NMR (300 MHz, CDCl₃): δ =1.74 (s, 3H), 2.31 (d, J =14 Hz, 1 H), 2.48 (d, J = 14 Hz, 1 H), 2.90 (s, 3H), 3.33 (s, 1 H), 3.99 (s, 3H), 4.58 (s, 1 H), 5.02 (s, 1 H), 5.27 (d, J = 7 Hz, 1H),
5.94 (d, J = 7 Hz, 1 H), 6.14 (dd, J = 3/8 Hz, 1 H), 6.90-6.95 (m, 2H), 7.22-7.28 (m,1 H), 7.36 (d, J = 8 Hz, 1 H), 9.42 (s, 1 H).
(-)-Enantiomer: Schmp.: 72-73°C; HPLC: Rₜ = 8.4 min (Chiralpak 10µ, 250x4.6 mm, Hex/EtOH 5%)
(+)-Enantiomer: Schmp.: 70-71°C; HPLC: Rₜ = 11.9 min (Chiralpak 10µ, 250x4.6 mm, Hex/EtOH 5%)

### Beispiel 8

### 2-{4-Brom-alpha-[(1H-indazol-4-yl)amino]-2-methoxybenzyl}-1,1,1-trifluor-4-methylpent-4-en-2-ol

¹H-NMR (300 MHz, CD₃OD): δ = 1.79 (3H), 2.28 (1 H), 2.52 (1 H), 4.00 (3H), 4.68 (1 H), 4.81 (1 H), 5.39 (1 H), 5.98 (1 H), 6.74 (1 H), 7.00-7.12 (2H), 7.19 (1 H), 7.45 (1 H), 8.12 (1 H)
und
2-{4-Brom-alpha-[(1H-indazol-4-yl)amino]-2-methoxybenzyl}-1,1,1-trifluor-4-methylpent-3-en-2-ol (SL 4753-3)
¹H-NMR (300 MHz, CD₃OD): δ = 1.39 (3H), 1.61 (3H), 3.98 (3H), 5.25 (1 H), 5.39 (1 H), 5.91 (1 H), 6.75 (1 H), 6.69-7.10 (2H), 7.10 (1 H), 7.42 (1 H), 8.13 (1 H)

### Beispiel 9

### 5-{[1-(4-Chlor-2-hydroxyphenyl)-2-hydroxy-4-methyl-2-(trifluormethyl)-pent-3-en-1-yl]amino}-isocoumarin

### 5-{([4-(4-Chlor-2-methoxyphenyl)-2-hydroxy-4-methyl-2-(trifluormethyl)pentyliden]amino}-isocoumarin

200 mg (0,616 mmol) 4-(4-Chlor-2-methoxyphenyl)-2-hydroxy-4-methyl-2-(trifluormethyl)-pentanal werden mit 99,3 mg (0,616 mmol) 5- Aminoisocoumarin (entstanden durch Reduktion des in Beispiel 3 beschriebenen 5-Nitroisocoumarins mit Zink und Ammoniumchlorid in EtOH, Tetrahydrofuran, Wasser) in 0,9 ml Eisessig versetzt und vier Tage bei Raumtemperatur gerührt. Das Reaktiongemisch wird dreimal mit Toluol abgezogen und der verbleibende Rückstand an Kieselgel (Laufmittel Ethylacetat/Hexan) chromatographiert. Isoliert werden 279,4 mg (96,9%) des gewünschten Imins.
¹H-NMR (300 MHz, CDCl₃): δ = 1.37 (3H), 1.58 (3H), 2.23 (1 H), 2.42 (1 H), 3.87 (3H), 4.64 (1 H), 6.50 (1 H), 6.10-6.19 (2H), 6.22 (1 H), 7.03 (1 H), 7.33 (1 H), 7.35-7.46 (2H), 8.20 (1 H)

### 5-{[1-(4-Chlor-2-hydroxyphenyl)-2-hydroxy-4-methyl-2-(trifluormethyl)-pent-3-en-1-yl]amino}-isocoumarin

270 mg (0,577 mmol) des oben beschriebenene lmins werden mit 5,7 ml einer 1 M Lösung von BBr₃ in Dichlormethan versetzt und zweieinhalb Stunden bei Raumtemperatur gerührt. Nach dem üblichen Aufarbeiten wird der Rückstand am Flashmaster chromatographiert
(NH₂ Säule, Laufmittel Methanol/Dichlormethan). Isoliert werden 38,6 mg der gewünschten Verbindung.
Smp.: 103-107°C

### Beispiel 10

### 2-{4-Fluor-alpha-[(2-methylchinazolin-5-yl)amino]-2-methoxybenzyl}-1,1,1-trifluor-4-methylpent-4-en-2-ol und 2-{4-Fluor-alpha-[(2-methylchinazolin-5-yl)amino]-2-methoxybenzyl}-1,1,1-trifluor-4-methylpent-3-en-2-ol

Analog Bsp. 6 werden 340 mg (1,1 mmol) 4-(4-Fluor-2-methoxyphenyl)-2-hydroxy-4-methyl-2-(trifluormethyl)pentanal und 211 mg (1.32 mmol) 5-Amino-2-methylchinazolin (beschrieben in Beispiel 6) mit 0.46 ml (2.2 mmol) Titantetraethylat zum lmin umgesetzt. Nach analoger Umlagerung mit Bortribromid und nachfolgender Chromatographie an Kieselgel mit Hexan-Essigester (0-50 %) werden 156 mg eines Gemisches aus 2-{4-Fluor-alpha-[(2-methylchinazolin-5-yl)amino]-2-methoxybenzyl}-1,1,1-trifluor-4-methylpent-4-en-2-ol und 2-14-Fluor-alpha-[(2-methylchinazolin-5-yl)amino]-2-methoxybenzyl}-1, 1, 1-trifluor-4-methylpent-3-en-2-01 erhalten. Die Trennung erfolgt mittels präparativer Dünnschichtchromatographie an Kieselgel (Dichlormethan 2-Propanol 5%) und liefert 51 mg der Hauptkomponente und 9 mg der Minderkomponente.
¹H-NMR (300 MHz, CDCl₃) der Hauptkomponente: δ = 1.74 (s, 3H), 2.33 (d, 1 H), 2.50 (d, 1 H), 2,85 (s, 3H), 3.41 (s, 1 H), 3.98 (s, 3H), 4.58 (s, 1 H), 5.02 (s, 1 H), 5.31 (d, 1 H), 6.12 (d, 1 H), 6.27 (d, 1 H), 6.60-6.71 (m, 2H), 7.17 (d, 1 H), 7.40 (dd, 1 H), 7.50 (t, 1 H),
9.39 (s, 1 H).
¹H-NMR (300 MHz, CDCl₃) der Minderkomponente: δ =1.45 (s, 3H), 1.64 (s, 3H), 2,75 (s, 3H),
3.92 (s, 3H), 5.31 (s, 1 H), 5.33 (d, 1 H), 6.23 (d, 1 H), 6.42 (d, 1 H), 6.54-6.64 (m, 2H), 7.15 (d, 1H), 7.46 (dd, 1 H), 7.53 (t, 1 H), 9.46 (s, 1 H).

### Beispiel 11

### 2-{4-Fluor-alpha-[(2-methylchinazolin-5-yl)aminol-2-methoxybenzyl}-1,1,1-trifluor-4-methylpentan-2-ol

17 mg (0.04 mmol) (rac.) 2-{4-Fluor-alpha-[(2-methylchinazolin-5-yl)amino]-2-methoxybenzyl}-1,1,1-trifluor-4-methylpent-4-en-2-ol werden in 2 ml Methanol, 2 ml Ethylacetat und 0.05 ml Triethylamin mit 20 mg Palladium auf Kohle (10%) 5 Stunden lang unter einer Wasserstoff Atmosphäre geschüttelt. Man versetzt die Reaktionsmischung mit 200 mg aktiviertem Mangandioxid und filtriert nach 10 Minuten durch Celite. Man engt ein und erhält nach präparativer Dünnschichtchromatographie an Kieselgel (Cyclohexan / Ethylacetat 50%) 3 mg gewünschtes Produkt.
¹H-NMR (300 MHz CDCl₃); δ = 0.83 (d, 3H), 0.91 (s, 3H), 1.78 (m, 1 H), 2,85 (s, 3H), 3.98 (s, 3H), 5.25 (d, 1 H), 6.10 (d, 1 H), 6.35 (d, 1 H), 6.63-6.72 (m, 2H), 7.19 (d, 1 H), 7.40 (dd, 1 H), 7.53 (t, 1 H), 9.40 (s, 1 H).

### Beispiel 12

### 2-{4-Chlor-5-fluor-alpha-[(2-methylchinazolin-5-yl)amino]-2-methoxybenzyl}-1,1,1-trifluor-4-methylpent-4-en-2-ol

Analog Bsp. 6 werden 200 mg (0.58 mmol) 4-(4-Chlor-5-fluor-2-methoxyphenyl)-2-hydroxy-4-methyl-2-(trifluormethyl)pentanal und 111 mg (0.70 mmol) 5-Amino-2-methylchinazolin mit 0.24 ml (1.16 mmol) Titantetraethylat zum lmin umgesetzt. Nach analoger Umlagerung mit Bortribromid und nachfolgender Chromatographie an Kieselgel (Dichlormethan/Methanol 5%) werden 34 mg der Titelverbindung erhalten.
¹H-NMR (300 MHz, CDCl₃); δ = 1.45 (s, 3H), 1.64 (s, 3H), 2,75 (s, 3H), 3.92 (s, 3H), 5.31 (s, 1 H), 5.33 (d, 1 H), 6.23 (d, 1 H), 6.42 (d, 1 H), 6.54-6.64 (m, 2H), 7.15 (d, 1 H), 7.46 (dd, 1 H), 7.53 (t, 1 H), 9.46 (s, 1 H).

### Beispiel 13

### 2-{4-Fluor-alpha-[(7-fluor-2-methylchinazolin-5-yl)amino]-2-methoxybenzyl}-1,1,1-trifluor-4-methylpent-3-en-2-ol

### 5-Amino-7-fluoro-2-methychinazolin

17 g (70,5 mmol) 3,6-Difluor-2-N-pivaloylaminobenzaldehyd (L. Florvall, I Fagervall, L.-G- Larsson, S.B. Ross, *Eur.J. Med. Chem.* **34** (1999) *137-151*), 9,2 g Acetamidinhydrochlorid, 13,4g Kaliumcarbonat und 10,4 g Molekularsieb (4A) werden in 70 ml Butyronitril zusammengegeben. Man erhitzt unter heftigem Rühren 17 Stunden auf 145°C und entfernt das Lösungsmittel im Vakuum. Nach Chromatographie des Rückstands an Kieselgel mit Hexan/Ethylacetat(0-70%) erhält man 4,5 g 7-Fluoro-5-N-pivaloylamino-2-methychinazolin.
1g (3,82 mmol) 7-Fluoro-5-N-pivaloylamino-2-methychinazolin werden in 74 ml Toluol geöst und auf 70°C gekühlt. Über 30 min werden 9,5 ml (11,4 mmol) einer 1,2 M Diisobutylaluminiumhydrid Lösung in Toluol zugetropft. Man lässt die Reaktionsmischung auf -40°C erwärmen und rührt 4 Stunden bei -40°C. Es wird langsam Wasser zugegeben und 30 Minuten bei Raumtemperatur gerührt bis sich ein Niederschlag bildet, der mittels Filtration durch Cellite entfernt wird. Man trennt die Phasen, wäscht mit gesättigter Natriumchlorid Lösung und trocknet über Natriumsulfat. Nach Chromatographie an Kieselgel mit Hexan-Essigester (0-100%) werden 64 mg des Produkts erhalten.
¹H-NMR (300 MHz, CDCl₃); δ = 2.83 (s, 3H), 4.67 (br., 2H), 6.50 (dd, 1 H), 6.93 (dd, 1 H), 9.23 (s, 1 H).

Zu 150 mg (0.48 mmol) 4-(4-Fluor-2-methoxyphenyl)-2-Hydroxy-4-methyl-2-(trifluormethyl)-pentanal und 85 mg (0.48 mmol) 5-Amino-7-fluor-2-methylchinazolin in 8 ml Toluol werden 0.25 ml Titantetraethylat gegeben und die Mischung wird über 2 Stunden auf 100°C erhitzt. Nach dem Abkühlen gießt man auf Wasser und rührt heftig nach. Die Suspension wird durch Celite filtriert, wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum. Die so roh erhaltenen 220 mg 4-(4-Fluor-2-methoxyphenyl)-1-(7-fluor-2-methylchinazolin-5-ylimino)-4-methyl-2-(trifluormethyl)-pentan-2-ol werden in 8 ml Dichlormethan aufgenommen und auf -70°C gekühlt. Man tropft über 10 Minuten 3 ml (3 mmol) einer 1 M Titantetrachlorid Lösung in Dichlormethan zu und lässt über 4 Stunden auf Raumtemperatur erwärmen. Man gießt die Lösung auf eine gesättigte Natriumhydrogencarbonat Lösung und rührt 5 Minuten lang heftig. Es wird mit Ethylacetat extrahiert, mit gesättigter Natriumchlorid Lösung gewaschen und über Natriumsulfat getrocknet. Nach Einengen und Chromatographie an Kieselgel (Dichlormethan / Methanol 10 %) erhält man 25 mg des gewünschten Produktes. ¹H-NMR (300 MHz, CDCl₃); δ =1.53 (s, 3H), 1.66 (s, 3H), 2.12 (d, 1 H), 2.27 (d, 1 H), 2,84 (s, 3H), 4.94 (d, 1 H), 5.99 (s, 1 H), 6.00 (s, 1 H), 6.02 (d, 1 H), 6.50 (dd, 1 H), 6.68 (d, 1 H), 6.83 (d, 1 H), 6.89 (dd, 1 H), 9.26 (s, 1 H).

### Beispiel 14

### 2-{6-Fluor-alpha-[(8-fluor-2-methylchinazolin-5-yl)amino]-2-hydroxybenzyl}-1,1,1-trifluor-4-methylpent-4-en-2-ol

Analog Bsp. 6 werden 200 mg ( 0.65 mmol) 4-(6-Fluor-2-methoxyphenyl)-2-hydroxy-4-methyl-2-(trifluormethyl)pentanal und 141 mg (0.80 mmol) 5-Amino-8-fluor-2-methylchinazolin (Herstellung beschrieben in Beispiel 7) mit 0.33 ml (1.58 mmol) Titantetraethylat zum Imin umgesetzt. Nach analoger Umlagerung mit Bortribromid und nachfolgender Chromatographie an Kieselgel (Hexan / Aceton 50%) werden 28 mg der Titelverbindung erhalten.
¹H-NMR (300 MHz, CDCl₃); δ =1.80 (s, 3H), 2.38 (d, 1 H), 2.55 (d, 1 H), 2,85 (s, 3H), 4.66 (s, 1 H), 4.95 (s, 1 H), 5.45 (d, 1 H), 6.34 (d, 1 H), 6.60-6.74 (m, 3H), 7.13 (m, 1 H), 7.46 (t, 1 H), 9.43 (s, 1 H).

### Beispiel 15

### 2-{4-Fluor-alpha-[(2-methylphthalazin-1-on-5-yl)amino]-2-hydroxybenzyl}-1,1,1-trifluor-4-methylpent-4-en-2-ol und 2-{4-Fluor-alpha-[(2-methylphthalazin-1-on-5-yl)amino]-2-hydroxybenzyl}-1,1,1-trifluor-4-methylpent-3-en-2-ol 3-Brom-4-nitro-phthalid

5.37 g 4-Nitrophthalid (Tetrahedron Lett. (2001), 42, pp. 1647-50), 8.04 g *N-*Bromsuccinimid und 196 mg Benzoylperoxyd werden in 80 ml Benzotrifluorid unter Rückfluß und Lichteinwirkung bis zur vollständigen Umsetzung erhitzt. Es wird auf Wasser gegeben, mit Dichlormethan extrahiert, mehrfach mit Wasser gewaschen, getrocknet und das Lösungsmittel im Vakuum entfernt. Man erhält 7.24 g 3-Brom-4-nitro-phthalid als Feststoff.
¹H-NMR (CDCl₃), δ (ppm) = 7.26 (s, 1 H), 7.88 (t, 1 H), 8.3 (d, 1 H), 8.56 (d, 1 H)

### 5-Nitro-phthalazin-1-on

18.25 g Hydrazinsulfat und 14.88 g Natriumcarbonat werden in 300 ml DMF bei 100°C für 1 h gerührt. Dann werden 7.24 g 3-Brom-4-nitro-phthalid in 100 ml DMF zugegeben und es wird weitere 4 h bei 100°C gerührt. Es wird auf Wasser gegeben, mit Ethylacetat mehrfach extrahiert und die organische Phase mit Wasser und Sole gewaschen. Es wird getrocknet und das Lösungsmittel im Vakuum entfernt. Nach Umkristallisation aus Ethylacetat erhält man 2.35 g 5-Nitro-phthalazin-1-on als Feststoff.
¹H-NMR (DMSO), δ (ppm) = 8.05 (t, 1 H), 8.57-8.66 (m, 2H), 8.73 (s, 1 H), 13.13 (bs, 1H)

### 2-Methy-5-nitro-phthalazin- 1-on

1.6 g 5-Nitro-phthalazin-1-on und 2.31 g Kaliumcarbonat werden 10 Minuten bei Raumtemperatur in 60 ml DMF gerührt. Es werden 1.1 ml Methyliodid zugegeben, und man rührt über Nacht. Es wird auf Wasser gegeben, mit Ethylacetat mehrfach extrahiert und die organische Phase mit Wasser und Sole gewaschen. Es wird getrocknet und das Lösungsmittel im Vakuum entfernt. Man erhält 1.57 g 2-Methy-5-nitro-phthalazin-1-on als gelben Feststoff.
¹H-NMR (DMSO), δ (ppm) = 3.73 (s, 3H), 8.05 (t, 1 H), 8.62 (d, 2H), 8.75 (s, 1 H)

### 5-Amino-2-methylphthalazin-1-on

1.57 g 2-Methy-5-nitrophthalazin-1-on und 130 mg Palladium auf Aktivkohle werden in 45 ml Ethylacetat suspendiert und mit Wasserstoff unter Normaldruck hydriert. Es wird durch Kieselgur filtriert und das Lösungsmittel im Vakuum entfernt. Man erhält 1.26 g 5-Amino-2-methylphthalazin-1-on als gelben Feststoff.
¹H-NMR (CDCl₃), δ (ppm) = 3.81 (s, 3H), 7.0 (d, 1 H), 7.5 (t, 1 H), 7.8 (d, 1 H), 8.16 (s, 1 H)

### 2-{4-Fluor-alpha-[(2-methylphthalazin-1-on-5-yl)amino]-2-hydroxybenzyl}-1,1,1-trifluor-4-methylpent-4-en-2-ol und 2-{4-Fluor-alpha-[(2-methylphthalazin-1-on-5-yl)amino]-2-hydroxybenryl}-1,1,1-trifluor-4-methylpent-3-en-2-ol

Analog Beispiel 6 werden 1.0 g 4-(4-Fluor-2-methoxyphenyl)-2-hydroxy-4-methyl-2-(trifluormethyl)pentanal und 560 mg 5-Amino-2-methylphthalazin-1-on mit 3.8 ml Titantetraethylat zum Imin umgesetzt. Nach analoger Umlagerung mit Bortribromid und nachfolgender HPLC Chromatographie werden 110 mg 2-{4-Fluor-alpha-[(2-methylphthalazin-1-on-5-yl)amino]-2-hydroxybenzyl}-1,1,1-trifluor-4-methylpent-4-en-2-ol als Hauptverbindung und 38 mg 2-{4-Fluor-alpha-[(2-methylphthalazin-1-on-5-yl)amino]-2-hydroxybenzyl}-1,1,1-trifluor-4-methylpent-3-en-2-ol als Nebenverbindung erhalten.
Hauptverbindung: ¹H-NMR (300 MHz, CD₃OD): δ = 1.79 (s, 3H), 2.32 (d, 1H), 2.56 (d, 1 H), 3.81 (s, 3H), 4.65 (s, 1 H), 4.81 (s, 1 H), 5.25 (s, 1 H), 6.50 (dd, 1 H), 6.57 (d, 1 H), 6.90 (d, 1 H), 7.36-7.55 (m, 3H), 8.52 (s, 1 H)
Nebenverbindung: ¹H-NMR (300 MHz, CD₃OD): δ = 1.47 (s, 3H), 1.63 (s, 3H), 3.81 (s, 3H), 5.29 (s, 1 H), 5.31 (s, 1 H), 6.48 (dd, 1 H), 6.54 (dd, 1 H), 6.81 (d, 1 H), 7.40-7.55 (m, 3H), 8.54 (s, 1H).

### Beispiele 16 und 17

### (-)-2-{4-Fluor-alpha-[(2-methylphthalazin-1-on-5-yl)amino]-2-hydroxybenzyl}-1,1,1-trifluor-4-methylpent-3-en-2-ol und (+)-2-{4-Fluor-alpha-[(2-methylphthalazin-1-on-5-yl)amino]-2-hydroxybenzyl}-1,1,1-trifluor-4-methylpent-3-en-2-ol

Trennung von (+/-)-2-{4-Fluor-alpha-[(2-methylphthalazin-1-on-5-yl)amino]-2-hydroxybenzyl}-1,1,1-trifluor-4-methylpent-3-en-2-ol
Das Enantiomerengemisch wird durch Chromatographie an chiralem Trägermaterial (CHIRALPAK AD^{®}, Fa DAICEL) mit Hexan / Ethanol (93 : 7, vvv) getrennt. Man erhält so das
(-)-Enantiomer: MS (EI): M⁺ = 451, [α]_{D} -169.8° (c = 1.0, CHCl₃) und das
(+)-Enantiomer: MS (EI): M⁺ = 451, [α]_{D} -146.3° (c =1.0, CHCl₃)

### Beispiel 18

### 2-{4-Brom-alpha-[(chinolin-2-on-5-yl)amino]-2-hydroxybenzyl}-1,1,1-trifluor-4-methylpent-4-en-2-ol und 2-{4-Brom-alpha-[(chinolin-2-on-5-yl)amino]-2-hydroxybenzyl}-1,1,1-trifluor-4-methylpent-3-en-2-ol

### 5-Aminochinolin-2(1H)-on

4.5 g 5-Nitrochinolin-2(1 H)-on (Chem. Pharm. Bull. (1981), 29, pp. 651-56) werden in 200 ml Essigester und 500 ml Methanol in Anwesenheit von 450 mg Palladium auf Aktivkohle als Katalysator unter Normaldruck mit Wasserstoff bis zur vollständigen Umsetzung hydriert. Der Katalysator wird durch Filtration durch Kieselgur entfernt und die Reaktionslösung im Vakuumeingeengt. Man erhält 3.8 g der Titelverbindung als gelben Feststoff.
¹H-NMR (DMSO): δ = 5.85 (bs, 2H), 6.27 (d, 1H), 6.33 (d, 1H), 6.43 (d, 1H), 7.10 (t, 1 H), 8.07 (d, 1 H), 11.39 (bs, 1 H)

Analog Beispiel 6 werden 800 mg 4-(4-Brom-2-methoxyphenyl)-2-hydroxy-4-methyl-2-(trifluormethyl)pentanal und 348 mg 5-Amino-chinolin-2-on mit 2.5 ml Titantetraethylat zum Imin umgesetzt. Nach analoger Umlagerung mit Bortribromid und nachfolgender Chromatographie an Kieselgel werden 53 mg 2-{4-Brom-alpha-[(Chinolin-2-on-5-yl)amino]-2-hydroxybenzyl}-1,1,1-trifluor-4-methylpent-4-en-2-ol (Fraktion A) und 54 mg 2-{4-Brom-alpha-[(chinolin-2-on-5-yl)amino]-2-hydroxybenzyl}-1,1,1-trifluor-4-methylpent-3-en-2-ol (Fraktion B) erhalten.
Fraktion A: ¹H-NMR (300 MHz, CD₃OD): δ =1.78 (s, 3H), 2.31 (d, 1 H), 2.59 (d, 1 H), 4.67 (s, 1 H), 4.82 (s, 1 H), 5.26 (s, 1 H), 6.29 (d, 1 H), 6.55 (d, 1 H), 6.62 (d, 1 H), 6.96 (dd, 1 H), 7.01 (d, 1 H), 7.22 (t, 1 H), 7.33 (d, 1 H), 8.21 (d, 1 H).
Fraktion B: ¹H-NMR (300 MHz, CD₃OD) δ = 1.46 (s, 3H), 1.62 (s, 3H), 5.28 (s, 1 H), 5.32 (d, 1 H), 6.23 (d, 1 H), 6.55 (d, 1 H), 6.60 (d, 1 H), 6.89 (dd, 1 H), 6.94 (d, 1 H), 7.21 (t, 1 H), 7.32 (d, 1 H), 8.22 (d, 1 H).

### Beispiel 19

### 4-Chlor-5-fluor-β-[(7-fluor-2-methylchinazolin-5-yl)aminol-2-methoxy-α-(2-methyl-2-propenyl)-α-(trifluormethyl)benzenethanol

Analog Beispiel 6 werden 200 mg (0.58 mmol) 4-(4-Chlor-5-fluor-2-methoxyphenyl)-2-hydroxy-4-methyl-2-(trifluormethyl)pentanal und 123 mg (0.70 mmol) 5-Amino-7-fluor-2-methylchinazolin mit 0.24 ml (1.16 mmol) Titantetraethylat zum Imin umgesetzt. Nach analoger Umlagerung mit Bortribromid und nachfolgender Chromatographie an Kieselgel (Hexan/Ethylacetat 33%) werden 51 mg der Titelverbindung erhalten.
¹H-NMR (CDCl₃); δ = 1.78 (s, 3H), 2.26 (d, 1 H), 2.52 (d, 1 H), 2.77 (s, 3H), 4.05 (s, 3H), 4.66 (s,1 H), 4.82 (s, 1 H), 5.33 (s, 1 H), 6.22 (dd, 1 H), 6.73 (dd, 1 H), 7.22 (d, 1 H), 7.62 (d, 1 H), 9.55 (s, 1 H).

### Beispiele 20 und 21

### (-)-4-Chlor-5-fluor-β-j(2-methylchinazolin-5-yl)amino]-a-(2-methyl-2-propenyl)-2-methoxy-α-(trifluormethyl)benzenethanol und (+)-4-Chlor-5-fluor-β-[(2-methylchinazolin-5-yl)amino]-α-(2-methyl-2-propenyl)-2-methoxy-α-(trifluormethyl)benzenethanol

(rac)- 4-Chlor-5-fluor-β-[(2-methylchinazolin-5-yl)amino]-α-(2-methyl-2-propenyl)-2-methoxy-α-(trifluormethyl)benzenethanol wird mittels präparativer chiraler HPLC (Chirapak AD 20µM) in die enanantiomerenreinen Verbindungen gespalten:
(-)-Enantiomer: analytische HPLC: Rₜ = 8.4 min (Chiralpak AD-H 5µ, 150x4.6 mm, Hexan / *iso*-Propanol 5%, 1 ml/min Fluß)
(+)-Enantiomer: analytische HPLC: Rₜ = 10.1 min (Chiralpak AD-5*µ*, 150x4.6 mm, Hexan / *iso*-Propanol 5%, 1 ml/min Fluß)

### Beispiel 22

### 3-Chlor-2-fluor-ß-[(7-fluor-2-methylchinazolin-5-yl)amino]-2-hydroxy-α-(2-methyl-1-propenyl)-α-(trifluormethyl)benzenethanol

312 mg (0,622 mmol) des wie üblich hergestellten lmins 4-(4-Chlor-3-fluor-2-methoxyphenyl)-1,1,1-trifluor-2-{[7-fluor-2-methylchinazolin-5-ylimino]-methyl}-4-methyl-pentan-2-ol werden mit 6,4 ml Bortribromid (1 M in Dichlormethan) versetzt und zwei Stunden bei Raumtemperatur gerührt. Nach dem üblichen Aufarbeiten und Chromatographieren am Flashmaster werden 1,6 mg (0,52 %) der gewünschten Verbindung erhalten.
¹H-NMR (CD₃OD): δ = 1,33 (3H), 1,62 (3H), 2,79 (3H), 5,09 (1H), 4,92 (1H, liegt unter dem Wasserpeak des Methanols), 6,43 (1 H), 6,50-6,70 (2H), 76,95 (1 H), 9,49 (1 H).

### Beispiele 23 und 24

### (-) 2-{4-Chlor-α-[(8-fluor-2-methylchinazolin-5-yl)amino]-2-methoxybenzyl}-1,1,1-trifluor-4-methylpent-3-en-2-oi und (+) 2-{4-Chlor-α-[(8-fluor-2-methylchinazolin-5-yl)amino]-2-methoxybenzyl}-1,1,1-trifluor-4-methylpent-3-en-2-ol

Das Beiprodukt *(rac.)* 2-{4-Chlor-α-[(8-fluor-2-methylchinazolin-5-yl)amino]-2-methoxybenzyl}-1,1,1-trifluor-4-methylpent-3-en-2-ol aus Beispiel 7 wird mit Hilfe der chiralen präparativen HPLC in seine Enantiomeren getrennt. Säule: Chiralpak AD 10*µ* (250 x 20 mm); Eluent Hexan / 7% 2-Propanol isokratisch; Fluß: 20 ml/min. Analytik: Chiralpak AD 10*µ* (205 x 4.6 mm); Eluent Hexan / 7% 2-Propanol isokratisch; Fluß: 1.0 ml/min; 25°C.
Das (-)-Enantiomer kommt bei einer Retentionszeit von Rₜ =10.35 min; spez. optische Drehung: - 278.3 (c = 0.230; CHCl₃). Das (+)-Enantiomer kommt bei einer Retentionszeit von Rₜ = 15.41 min.

### Beispiele 25 und 26

### (-)-2-{4-Fluor-alpha-[(2-methylphthalazin-1-on-5-yl)amino]-2-hydroxybenzyl}-1,1,1-trifluor-4-methylpent-4-en-2-ol und (+)-2-{4-Fluor-alpha-[(2-methylphthalazin-1-on-5-yl)amino]-2-hydroxybenzyl}-1,1,1-trifluor-4-methylpent-4-en-2-ol

Trennung von (+/-)-2-(4-Fluor-alpha-[(2-methylphthalazin-1-on-5-yl)amino]-2-hydroxybenzyl}-1,1,1-trifluor-4-methylpent-4-en-2-ol
Das Enantiomerengemisch wird durch Chromatographie an chiralem Trägermaterial (CHIRALPAK AD^{®}, Fa DAICEL) mit Hexan / Ethanol (93 : 7, vvv) getrennt. Man erhält so das
(-)-Enantiomer: MS (EI): M⁺ = 451, [α]_{D} -224.3° (c = 1.0, CHCl₃) und das
(+)-Enantiomer: MS (EI): M⁺ = 451, [α]_{D} +207.6 (c = 1.0, CHCl₃)

### Beispiel 27

### 2-{4-Chlor-alpha-[(phthalazin-1-on-5-yl)amino]-2-methoxybenzyl}-1,1,1-trifluor-4-methylpent-4-en-2-ol und 2-{4-Chlor-alpha-[(phthalazin-1-on-5-yl)amino]-2-methoxybenzyl}-1,1,1-trifluor-4-methylpent-3-en-2-ol

### 5-Amino-phthalazin-1-on

980 mg 5-Nitro-phthalazin-1-on (Beispiel 66) und 100 mg Palladium auf Aktivkohle werden in 50 ml Essigester und 1 ml Triethylamin suspendiert und mit Wasserstoff unter Normaldruck hydriert. Es wird durch Kieselgur filtriert und das Lösungsmittel im Vakuum entfernt. Man erhält als Rohprodukt 830 mg 5-Amino-phthalazin-1-on als Feststoff.
¹H-NMR (DMSO), δ (ppm) = 6.26 (bs, 2H), 7.00 (d, 1 H), 7.32 (d, 1 H), 7.44 (t, 1 H), 8.48 (s, 1 H), 12.35 (bs, 1 H)

Analog Beispiel 6 werden 500 mg 4-(4-Chlor-2-methoxyphenyl)-2-hydroxy-4-methyl-2-(trifluormethyl)pentanal und 250 mg 5-Amino-phthalazin-1-on mit 1.8 ml Titantetraethylat zum Imin umgesetzt. Nach analoger Umlagerung mit Bortribromid und nachfolgender Chromatographie an Kieselgel werden 38 mg 2-(4-Chlor-alpha-[(phthalazin-1-on-5-yl)amino]-2-methoxybenzyl)-1,1,1-trifluor-4-methylpent-4-en-2-ol (Fraktion A) und 47 mg 2-{4-Chlor-alpha-[(phthalazin-1-on-5-yl)amino]-2-methoxybenzyl}-1,1,1-trifluor-4-methylpent-3-en-2-ol (Fraktion B) erhalten.
Fraktion A: ¹H-NMR (300 MHz, CD₃OD): δ = 1.80 (s, 3H), 2.27 (d, 1 H), 2.52 (d, 1 H), 4.04 (s, 3H), 4.67 (s, 1 H), 4.84 (s, 1 H), 5.35 (s, 1 H), 6.80 (d, 1 H), 6.97 (dd, 1 H), 7.11 (d, 1 H), 7.47-7.63 (m, 3H), 8.55 (s, 1 H).
Fraktion B: ¹H-NMR (300 MHz, CD₃OD): δ = 1.37 (s, 3H), 1.62 (s, 3H), 3.98 (s, 3H), 5.25 (s, 1 H), 5.32 (s, 1 H), 6.70 (d, 1 H), 6.91 (dd, 1 H), 7.04 (d, 1 H), 7.44-7.62 (m, 3H), 8.57 (s, 1 H).

### Beispiel 28

### 5-{[4-Chlor-5-fluor-2-methoxy-α-(2-methyl-1-propenyl)-α-(trifluormethyl)benzenethanol-β-yl]amino}-2-methylphthalazin-1-on

Analog Beispiel 6 werden 260 mg (0.76 mmol) 4-(4-Chlor-5-fluor-2-methoxyphenyl)-2-hydroxy-4-methyl-2-(trifluormethyl)pentanal und 160 mg (0.91 mmol) 5-Amino-2-methyl-phthalazin-1-on mit 0.3 ml (1.5 mmol) Titantetraethylat zum lmin umgesetzt. Nach analoger Umlagerung mit Bortribromid und nachfolgender Chromatographie an Kieselgel (Hexan/Ethylacetat 0-50%) werden 70 mg der Titelverbindung erhalten.
¹H-NMR (CDCl₃); δ = 1.64 (s, 3H), 1.67 (s, 3H), 3.84 (s, 3H), 3.92 (s, 3H), 5.20 (s, 1H), 5.24 (br, 1 H), 5.69 (br, 1 H), 6.46 (d, 1 H), 6.89 (d, 1 H), 7.22 (d, 1 H), 7.35 (t, 1 H), 7.63 (d, 1 H), 8.19 (s, 1H).

## Patentansprüche

1. Stereoisomere der allgemeinen Formel I 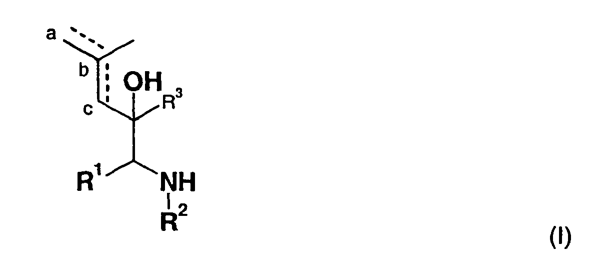 worin
R¹ einen gegebenenfalls substituierten Phenylrest,
R² einen mono- oder bizyklisches aromatisches, teilaromatisches oder nicht aromatisches Ringsystem, das gegebenenfalls 1-3 Stickstoffatome, 1-2 Sauerstoffatome und/oder 1-2 Schwefelatome enthält und gegebenenfalls ein- oder mehrfach substituiert ist durch einen Rest ausgewählt aus der Gruppe Carbonyl, Halogen, Hydroxy, (C₁-C₅)-Alkyl, der gegebenenfalls substituiert sein kann durch 1-3 Hydroxygruppen,1-3 (C₁-C₅)Alkoxygruppen und/oder 1-3 COOR⁶⁻Gruppen,
(C₁-C₅)Alkoxy, (C₁-C₅)-Alkylthio, (C₁-C₅)-Perfluoralkyl, Cyano, Nitro, oder zwei Substituenten zusammen eine Gruppe ausgewählt aus den Gruppen -O-(CH₂)ₙ-O-, -O-(CH₂)ₙ-CH₂-, -O-CH=CH-, -(CH₂)ₙ₊₂-, -NH-(CH₂)ₙ₊₁-, -N(C₁-C₃-alkyl)-(CH₂)ₙ₊₁-, -NH-N=CH-, bilden
wobei n = 1 oder 2 ist und die endständigen Sauerstoffatome und/oder Kohlenstoffatome und/oder Stickstoffatome mit direkt benachbarten Ring-Kohlenstoffatomen verknüpft sind, NR⁴R⁵
wobei R⁴ und R⁵ unabhängig voneinander Wasserstoff, C₁-C₅-Alkyl oder (CO)-C₁-C₅-Alkyl sein können, COOR⁶,
wobei R⁶ Wasserstoff oder eine C₁-C₅-Alkylgruppe bedeuten, (CO)NR⁷R⁸,
wobei R⁷ und R⁸ unabhängig voneinander Wasserstoff oder eine C₁-C₅₋Alkylgruppe bedeuten,
oder eine (C₁-C₅-Alkylen)-O-(CO)-(C₁-C₅)alkylgruppe
R³ eine C₁-C₃-Alkylgruppe oder eine teilweise oder vollständig fluorierte C₁₋C₃-Alkylgruppe
bedeuten,
worin der Phenylrest R¹ gegebenenfalls substituiert ist durch einen oder mehrere Reste aus der Gruppe C₁-C₅-Alkyl, C₁-C₅-Alkoxy, C₁-C₅-Alkylthio, C₁-C₅₋Perfluoralkyl, Halogen, Hydroxy, Cyano, Nitro, -O-(CH₂)ₙ-O-, -O-(CH₂)ₙ-CH₂-, -O-CH=CH-, -(CH₂)ₙ₊₂-, -NH-(CH₂)ₙ₊₁ , N(C₁-C₃₋alkyl)-(CH₂)ₙ₊₁, -NH-N=CH-,
wobei n = 1 oder 2 ist und die endständigen Sauerstoffatome und/oder Kohlenstoffatome und/oder Stickstoffatome mit direkt benachbarten Ring-Kohlenstoffatomen verknüpft sind,
oder NR⁴R⁵, wobei R⁴ und R⁵ wie zuvor definiert sind,
die gestrichelte Linie eine Doppelbindung zwischen den Atomen a und b oder eine Doppelbindung zwischen den Atomen b und c oder nur eine Einfachbindung zwischen den Atomen a und b sowie b und c bedeutet, sowie deren Racemate oder getrennt vorliegenden Stereoisomeren, und gegebenenfalls deren physiologisch verträgliche Salze.

2. Stereoisomere der allgemeinen Formel I 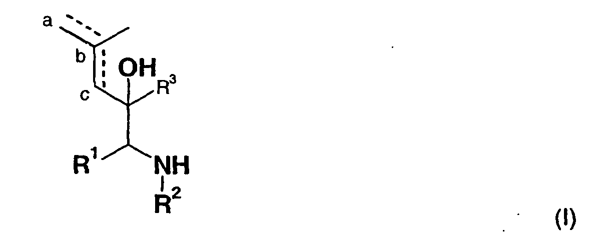 worin
R¹ einen gegebenenfalls substituierten Phenylrest,
R² einen mono- oder bizyklisches aromatisches, teilaromatisches oder nicht aromatisches Ringsystem, das gegebenenfalls 1-3 Stickstoffatome, 1-2 Sauerstoffatome und/oder 1-2 Schwefelatome enthält und gegebenenfalls ein- oder mehrfach substituiert ist durch einen Rest ausgewählt aus der Gruppe Carbonyl, Halogen, Hydroxy, (C₁-C₅)-Alkyl, der gegebenenfalls substituiert sein kann durch 1-3 Hydroxygruppen, 1-3 (C₁-C₅)Alkoxygruppen und/oder 1-3 COOR⁶⁻Gruppen,
(C₁-C₅)Alkoxy, (C₁-C₅)-Alkylthio, (C₁-C₅)-Perfluoralkyl, Cyano, Nitro, oderX
wobei R⁴ und R⁵ unabhängig voneinander Wasserstoff, C₁-C₅-Alkyl oder (CO)-C₁-C₅-Alkyl sein können, COOR⁶,
wobei R⁶ Wasserstoff oder eine C₁-C₅-Alkylgruppe bedeuten, (CO)NR⁷R⁸,
wobei R⁷ und R⁸ unabhängig voneinander Wasserstoff oder eine C₁-C₅₋Alkylgruppe bedeuten,
oder eine (C₁-C₅-Alkylen)-O-(CO)-(C₁-C₅)alkylgruppe
R³ eine C₁-C₃-Alkylgruppe oder eine teilweise oder vollständig fluorierte C₁₋C₃-Alkylgruppe
bedeuten,
die gestrichelte Linie eine Doppelbindung zwischen den Atomen a und b oder eine Doppelbindung zwischen den Atomen b und c oder nur eine Einfachbindung zwischen den Atomen a und b sowie b und c bedeutet, sowie deren Racemate oder getrennt vorliegenden Stereoisomeren, und gegebenenfalls deren physiologisch verträgliche Salze.

3. Stereoisomere gemäß Anspruch 1, worin der Phenylrest substituiert ist durch einen oder mehrere Reste aus der Gruppe C₁-C₅-Alkyl, C₁-C₅₋Alkoxy, C₁-C₅-Alkylthio, C₁-C₅-Perfluoralkyl, Halogen, Hydroxy, Cyano, Nitro,
-O-(CH₂)ₙ-O-, -O-(CH₂)ₙ-CH₂-, -O-CH=CH-, -(CH₂)ₙ₊₂-, -NH-(CH₂)ₙ₊₁, N(C₁-C₃-alkyl)-(CH₂)ₙ₊₁, -NH-N=CH-,
wobei n = 1 oder 2 ist und die endständigen Sauerstoffatome und/oder Kohlenstoffatome mit direkt benachbarten Ring-Kohlenstoffatomen verknüpft sind,
oder NR⁴R⁵,
wobei R⁴ und R⁵ unabhängig voneinander Wasserstoff, C₁-C₅-Alkyl oder (CO)-C₁-C₅-Alkyl sein können.

4. Stereoisomere gemäß Anspruch 1, worin R² eine über eine beliebige Position verknüpfte gegebenenfalls substituierte Phthalidyl-, Isoindolyl-, Dihydroindolyl-, Dihydroisoindolyl-, Dihydroisochinolinyl-, Thiophthalidyl-, Benzoxazinonyl-, Phthalazinonyl-, Chinolinyl-, Isochinolinyl-, Chinolonyl-, lsochinolonyl-, Indazolyl-, Benzothiazolyl-, Chinazolinyl-, Chinoxalinyl-, Cinnolinyl-, Phthalazinyl-, 1,7- oder 1,8-Naphthyridinyl-, Dihydroindolonyl-, Dihydroisoindolonyl-, Benzimidazol- oder Indolylgruppe bedeutet.

5. Verbindungen gemäß Anspruch 1, worin R³ für einen Trifluormethyl- oder Pentafluorethylrest steht.

6. Verwendung der Verbindungen gemäß Anspruch 1 zur Herstellung von Arzneimitteln.

7. Verwendung der Verbindungen gemäß Anspruch 1 zur Herstellung von Arzneimitteln zur Behandlung von entzündlichen Erkrankungen.

8. Stereoisomere gemäß Anspruch 1, worin die Phthalidyl-, lsoindolyl-, Dihydroindolyl-, Dihydroisoindolyl-, Dihydroisochinolinyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Cumarinyl, Isocumarinyl-, Phthalazinyl-, 1,7- oder 1,8-Naphthyridinyl-, Dihydroindolonyl-, Dihydroisoindolonyl-, Benzimidazol- oder Indolylgruppe R² mit 0 bis 3 gleichen oder verschiedenen Resten aus der Gruppe C₁-C₃-Alkyl, Hydroxy, Carbonyl oder Halogen substituiert sind.

9. Stereoisomere gemäß Anspruch 7, worin die Phthalidyl-, Isoindolyl-, Dihydroindolyl-, Dihydroisoindolyl-, Dihydroisochinolinyl-, Thiophthalidyl-, Benzoxazinonyl-, Phthalazinonyl-, Chinolinyl-, Isochinolinyl-, Chinolonyl-, Isochinolonyl-, Indazolyl-, Benzothiazolyl-, Chinazolinyl-, Chinoxalinyl-, Cinnolinyl-, Phthalazinyl-, 1,7- oder 1,8-Naphthyridinyl-, Dihydroindolonyl-, Dihydroisoindolonyl-, Benzimidazol- oder Indolylgruppe R² mit Methyl, Chlor oder Fluor substituiert ist.

10. Verfahren zur Herstellung der Verbindungen der allgemeinen Formel l, **dadurch gekennzeichnet, dass** Imine der allgemeinen Formel II, 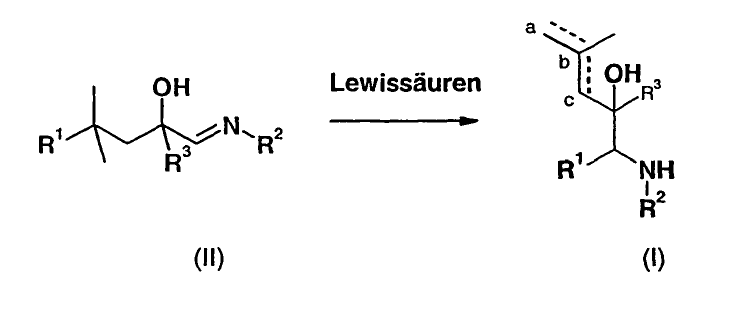 worin R¹, R² und R³ die in Anspruch 1 angegebenen Bedeutungen haben, mit Lewissäure in einem organischen Lösungsmittel, umgesetzt werden und Verbindungen der allgemeinen Formel I nach üblicher Aufreinigung erhalten werden.

## Claims

1. Stereoisomers of general formula I in which 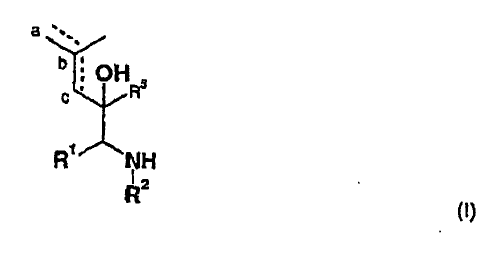 R¹ means an optionally substituted phenyl radical,
R² means a monocyclic, or bicyclic, aromatic, partially aromatic, or non-aromatic ring system, which optionally contains 1-3 nitrogen atoms, 1-2 oxygen atoms and/or 1-2 sulfur atoms and optionally is substituted in one or more places by a radical that is selected from the group carbonyl, halogen, hydroxy, or (C₁-C₅)-alkyl, which optionally can be substituted by 1-3 hydroxy groups, 1-3 (C₁-C₅)alkoxy groups and/or 1-3 COOR⁶ groups,
(C₁-C₅)alkoxy, (C₁-C₅)-alkylthio, (C₁-C₅) - perfluoroalkyl, cyano, nitro, or two substituents together form a group that is selected from the groups -O-(CH₂)ₙ-O-, -O-(CH₂)ₙ-CH₂-, -O-CH=CH-, - (CH₂)ₙ₊₂-, -NH- (CH₂)ₙ₊₁-, -N (C₁-C₃-alkyl) - (CH₂)ₙ₊₁-, and -NH-N=CH-,
wherein n=1 or 2, and the terminal oxygen atoms and/or carbon atoms and/or nitrogen atoms are linked to directly adjacent ring-carbon atoms, NR⁴R⁵,
wherein R⁴ and R⁵, independently of one another, can be hydrogen, C₁-C₅-alkyl or (CO)-C₁-C₅-alkyl, COOR⁶, wherein R⁶ means hydrogen or a C₁-C₅-alkyl group, (CO)NR⁷R⁸,
wherein R⁷ and R⁸, independently of one another, mean hydrogen or a C₁-C₅-alkyl group,
or a (C₁-C₅-alkylene)-O-(CO)-(C₁-C₅)alkyl group,
R³ means a C₁-C₃-alkyl group or a partially or completely fluorinated C₁-C₃-alkyl group,
in which the phenyl radical R¹ is optionally substituted by one or more radicals from the group C₁-C₅-alkyl, C₁-C₅-alkoxy, C₁-C₅-alkylthio, C₁-C₅-perfluoroalkyl, halogen, hydroxy, cyano, nitro, -O-(CH₂)ₙ-O-, -O-(CH₂)ₙ-CH₂-, -O-CH=CH-, -(CH₂)ₙ₊₂-, -NH-(CH₂)ₙ₊₁, N(C₁-C₃-alkyl)-(CH₂)ₙ₊₁, or -NH-N=CH-,
wherein n= or 2, and the terminal oxygen atoms and/or carbon atoms and/or nitrogen atoms are linked to directly adjacent ring-carbon atoms, or NR⁴R^{5,}
wherein R⁴ and R⁵ are as defined above,
and the dashed line means a double bond between atoms a and b or a double bond between atoms b and c or only a single bond between atoms a and b, as well as b and c, as well as their racemates or separately present stereoisomers, and optionally their physiologically compatible salts.

2. Stereoisomers of general formula I 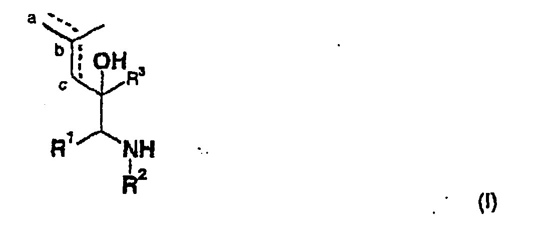 in which R¹ means an optionally substituted phenyl radical,
R² means a monocyclic or bicyclic aromatic, partially aromatic or non-aromatic ring system, which optionally contains 1-3 nitrogen atoms, 1-2 oxygen atoms and/or 1-2 sulfur atoms and optionally is substituted in one or more places by a radical that is selected from the group carbonyl, halogen, hydroxy, (C₁-C₅)-alkyl, which optionally can be substituted by 1-3 hydroxy groups, 1-3 (C₁-C₅)alkoxy groups and/or 1-3 COOR⁶ groups,
(C₁-C₅)alkoxy, (C₁-C₅)-alkylthio, (C₁-C₅)-perfluoroalkyl, cyano, nitro, or NR⁴R⁵,
wherein R⁴ and R⁵, independently of one another, can be hydrogen, C₁-C₅-alkyl or (CO)-C₁-C₅-alkyl, COOR⁶, wherein R⁶ means hydrogen or a C₁-C₅-alkyl group, (CO) NR⁷R⁸,
wherein R⁷ and R⁸, independently of one another, mean hydrogen or a C₁-C₅-alkyl group, or a (C₁-C₅₋alkylene)-O-(CO)-(C₁-C₅)alkyl group,
R³ means a C₁-C₃-alkyl group or a partially or completely fluorinated C₁-C₃-alkyl group,
and the dashed line means a double bond between atoms a and b or a double bond between atoms b and c or only a single bond between atoms a and b as well as b and c, as well as their racemates or separately present stereoisomers and optionally their physiologically compatible salts.

3. Stereoisomers according to claim 1, in which the phenyl radical is substituted by one or more radicals from the group C₁-C₅-alkyl, C₁-C₅-alkoxy, C₁-C₅-alkylthio, C₁-C₅-perfluoroalkyl, halogen, hydroxy, cyano, nitro, -O-(CH₂)ₙ-O-, -O-(CH₂)ₙ-CH₂-, -O-CH=CH-, -(CH₂)ₙ₊₂-, -NH-(CH₂)ₙ₊₁, N(C₁-C₃-alkyl)-(CH₂)ₙ₊₁, or -NH-N=CH-, wherein n=1 or 2, and the terminal oxygen atoms and/or carbon atoms are linked with directly adjacent ring-carbon atoms, or NR⁴ R⁵, wherein R⁴ and R⁵, independently of one another, can be hydrogen, C₁-C₅-alkyl or (CO) -C₁-C₅₋alkyl.

4. Stereoisomers according to claim 1, in which R² means an optionally substituted phthalidyl, isoindolyl, dihydroindolyl, dihydroisoindolyl, dihydroisoquinolinyl, thiophthalidyl, benzoxazinonyl, phthalazinonyl, quinolinyl, isoquinolinyl, quinolonyl, isoquinolonyl, indazolyl, benzothiazolyl, quinazolinyl, quinoxalinyl, cinnolinyl, phthalazinyl, 1,7- or 1,8-naphthyridinyl, dihydroindolonyl, dihydroisoindolonyl, benzimidazole or indolyl group that is linked via any position.

5. Compounds according to claim 1, in which R³ stands for a trifluoromethyl or pentafluoroethyl radical.

6. Use of the compounds according to claim 1 for the production of pharmaceutical agents.

7. Use of the compounds according to claim 1 for the production of pharmaceutical agents for treating inflammatory diseases.

8. Stereoisomers according to claim 1, in which the phthalidyl, isoindolyl, dihydroindolyl, dihydroisoindolyl, dihydroisoquinolinyl, thiophthalidyl, benzoxazinonyl, phthalazinonyl, quinolinyl, isoquinolinyl, quinolonyl, isoquinolonyl, indazolyl, benzothiazolyl, quinazolinyl, quinoxalinyl, cinnolinyl, coumarinyl, isocoumarinyl, phthalazinyl, 1,7- or 1,8-naphthyridinyl, dihydroindolonyl, dihydroisoindolonyl, benzimidazole or indolyl group R² is substituted with 0 to 3 of the same or different radicals from the group C₁-C₃-alkyl, hydroxy, carbonyl or halogen.

9. Stereoisomers according to claim 7, in which the phthalidyl, isoindolyl, dihydroindolyl, dihydroisoindolyl, dihydroisoquinolinyl, thiophthalidyl, benzoxazinonyl, phthalazinonyl, quinolinyl, isoquinolinyl, quinolonyl, isoquinolonyl, indazolyl, benzothiazolyl, quinazolinyl, quinoxalinyl, cinnolinyl, phthalazinyl, 1,7- or 1,8-naphthyridinyl, dihydroindolonyl, dihydroisoindolonyl, benzimidazole or indolyl group R² is substituted with methyl, chlorine or fluorine.

10. Process for the production of compounds of general formula I, **characterized in that** imines of general formula II 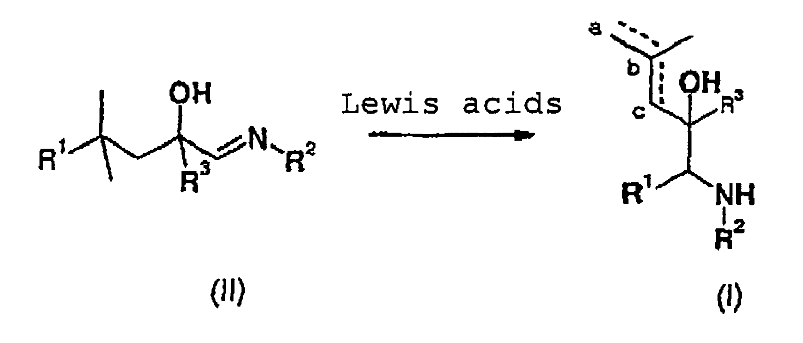 in which R¹, R² and R³ have the meanings that are indicated in claim 1, are reacted with Lewis acid in an organic solvent, and compounds of general formula I are obtained after conventional purification.

## Revendications

1. Stéréo-isomères de formule générale I 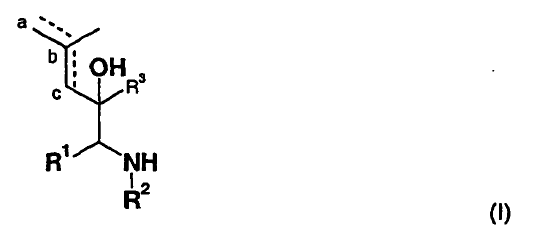 dans laquelle
R¹ signifie un radical phényle le cas échéant substitué, R² signifie un système cyclique monocyclique ou bicyclique, aromatique, partiellement aromatique ou non aromatique, qui contient le cas échéant 1-3 atomes d'azote, 1-2 atomes d'oxygène et/ou 1-2 atomes de soufre et qui est le cas échéant monosubstitué ou polysubstitué par un radical choisi dans le groupe formé par carbonyle, halogène, hydroxy, (C₁-C₅)-alkyle, qui peut le cas échéant être substitué par 1-3 groupes hydroxy, 1-3 groupes (C₁-C₅)alcoxy et/ou 1-3 groupes COOR⁶,
(C₁-C₅) alcoxy, (C₁-C₅) -alkylthio, (C₁-C₅)-perfluoroalkyle, cyano, nitro, ou deux substituants forment ensemble un groupe choisi parmi les groupes -O-(CH₂) ₙ-O-, -O- (CH₂) ₙ-CH₂-, -O-CH=CH-₁ - (CH₂) ₙ₊₂-, -NH (CH₂) ₙ₊₁-, -N (C₁-C₃-alkyl) - (CH₂)ₙ₊₁-, -NH-N=CH-
où n = 1 ou 2 et les atomes d'oxygène et/ou de carbone et/ou d'azote en position terminale sont liés à des atomes de carbone du cycle directement adjacents, NR₄R₅
où R⁴ et R⁵ peuvent représenter, indépendamment l'un de l'autre, hydrogène, C₁-C₅-alkyle ou (CO) -C₁-C₅-alkyle, COOR⁶,
où R⁶ signifie hydrogène ou un groupe C₁-C₅-alkyle, (CO) NR⁷R⁸,
où R⁷ et R⁸ signifient indépendamment l'un de l'autre hydrogène ou un groupe C₁-C₅-alkyle,
ou un groupe (C₁-C₅-alkylène) -O- (CO) - (C₁-C₅) alkyle
R³ signifie un groupe C₁-C₃-alkyle ou un groupe C₁-C₃₋alkyle partiellement ou totalement fluoré
où le radical phényle R¹ est le cas échéant substitué par un ou plusieurs radicaux du groupe formé par C₁-C₅₋alkyle, C₁-C₅-alcoxy, C₁-C₅-alkylthio, C₁-C₅₋perfluoroalkyle, halogène, hydroxy, cyano, nitro, -O-(CH₂)ₙ-O-, -0- (CH₂)ₙ-CH₂-, -O-CH=CH-, - (CH₂)ₙ₊₂-, -NH-(CH₂)ₙ₊₁, N(C₁-C₃-alkyl)-(CH₂)ₙ₊₁, -NH-N=CH-,
où n = 1 ou 2 et les atomes d'oxygène et/ou de carbone et/ou d'azote en position terminale sont liés à des atomes de carbone du cycle directement adjacents,
ou NR₄R₅, R⁴ et R⁵ étant définis comme ci-dessus,
la ligne en pointillés signifie une double liaison entre les atomes a et b ou une double liaison entre les atomes b et c ou seulement une simple liaison entre les atomes a et b ainsi que b et c, ainsi que leurs racémates ou stéréo-isomères se trouvant sous forme séparée et le cas échéant leurs sels physiologiquement acceptables.

2. Stéréo-isomères de formule générale 1 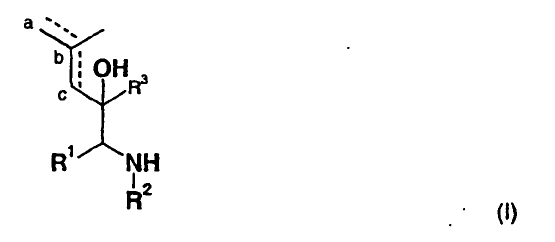 dans laquelle
R¹ signifie un radical phényle le cas échéant substitué, R² signifie un système cyclique monocyclique ou bicyclique, aromatique, partiellement aromatique ou non aromatique, qui contient le cas échéant 1-3 atomes d'azote, 1-2 atomes d'oxygène et/ou 1-2 atomes de soufre et qui est le cas échéant monosubstitué ou polysubstitué par un radical choisi dans le groupe formé par carbonyle, halogène, hydroxy, (C₁-C₅)-alkyle, qui peut le cas échéant être substitué par 1-3 groupes hydroxy, 1-3 groupes (C₁-C₅)alcoxy et/ou 1-3 groupes COOR⁶,
(C₁-C₅) alcoxy, (C₁-C₅)-alkylthio, (C₁-C₅) - perfluoroalkyle, cyano, nitro, ou NR⁴R⁵
où R⁴ et R⁵ peuvent représenter, indépendamment l'un de l'autre, hydrogène, C₁-C₅-alkyle ou (CO)-C₁-C₅-alkyle, COOR⁶,
où R⁶ signifie hydrogène ou un groupe C₁-C₅-alkyle, (CO)NR⁷R⁸,
où R⁷ et R⁸ signifient indépendamment l'un de l'autre hydrogène ou un groupe C₁-C₅-alkyle,
ou un groupe (C₁-C₅-alkylène) -O- (CO) - (C₁-C₅) alkyle
R³ signifie un groupe C₁-C₃-alkyle ou un groupe C₁-C₃₋alkyle partiellement ou totalement fluoré
la ligne en pointillés signifie une double liaison entre les atomes a et b ou une double liaison entre les atomes b et c ou seulement une simple liaison entre les atomes a et b ainsi que b et c, ainsi que leurs racémates ou stéréo-isomères se trouvant sous forme séparée et le cas échéant leurs sels physiologiquement acceptables.

3. Stéréo-isomères selon la revendication 1, où le radical phényle est substitué par un ou plusieurs radicaux du groupe formé par C₁-C₅-alkyle, C₁-C₅-alcoxy, C₁-C₅-alkylthio, C₁-C₅-perfluoroalkyle, halogène, hydroxy, cyano, nitro, -O-(CH₂)ₙ-O-, -O-(CH₂)ₙ-CH₂-, -O-CH=CH-, - (CH₂)ₙ₊₂-, -NH-(CH₂)ₙ₊₁, N(C₁-C₃-alkyl)-(CH₂)ₙ₊₁, -NH-N=CH-,
où n = 1 ou 2 et les atomes d'oxygène et/ou de carbone en position terminale sont liés avec des atomes de carbone du cycle directement adjacents,
ou NR⁴R⁵,
où R⁴ et R⁵ peuvent représenter, indépendamment l'un de l'autre, hydrogène, C₁-C₅-alkyle ou (CO)-C₁-C₅-alkyle,

4. Stéréo-isomères selon la revendication 1, où R² signifie un groupe phtalidyle, isoindolyle, dihydroindolyle, dihydroisoindolyle, dihydroisoquinoléinyle, thiophtalidyle, benzoxazinonyle, phtalazinonyle, quinoléinyle, isoquinoléinyle, quinolonyle, isoquinolonyle, indazolyle, benzothiazolyle, quinazolinyle, quinoxalinyle, cinnolinyle, phtalazinyle, 1,7-naphtyridinyle ou 1,8-naphtyridinyle, dihydroindolonyle, dihydroisoindolonyle, benzimidazole ou indolyle, lié via une position quelconque, le cas échéant substitué.

5. Composés selon la revendication 1, où R³ représente un radical trifluorométhyle ou pentafluoroéthyle.

6. Utilisation des composés selon la revendication 1 pour la préparation de médicaments.

7. Utilisation des composés selon la revendication 1 pour la préparation de médicaments destinés au traitement de maladies inflammatoires.

8. Stéréo-isomères selon la revendication 1, dans lesquels les groupes phtalidyle, isoindolyle, dihydroindolyle, dihydroisoindolyle, dihydroisoquinoléinyle, thiophtalidyle, benzoxazinonyle, phtalazinonyle, quinoléinyle, isoquinoléinyle, quinolonyle, isoquinolonyle, indazolyle, benzothiazolyle, quinazolinyle, quinoxalinyle, cinnolinyle, coumarinyle, isocoumarinyle, phtalazinyle, 1,7-naphtyridinyle ou 1,8-naphtyridinyle, dihydroindolonyle, dihydroisoindolonyle, benzimidazole ou indolyle R² sont substitués par 0 à 3 radicaux identiques ou différents du groupe C₁-C₃-alkyle, hydroxy, carbonyle ou halogène.

9. Stéréo-isomères selon la revendication 7, dans lesquels le groupe phtalidyle, isoindolyle, dihydroindolyle, dihydroisoindolyle, dihydroisoquinoléinyle, thiophtalidyle, benzoxazinonyle, phtalazinonyle, quinoléinyle, isoquinoléinyle, quinolonyle, isoquinolonyle, indazolyle, benzothiazolyle, quinazolinyle, quinoxalinyle, cinnolinyle, phtalazinyle, 1,7-naphtyridinyle ou 1,8-naphtyridinyle, dihydroindolonyle, dihydroisoindolonyle, benzimidazole ou indolyle R² est substitué par méthyle, chlore ou fluor.

10. Procédé de préparation de composés de formule générale I, **caractérisé en ce qu'**on transforme des imines de formule générale II, 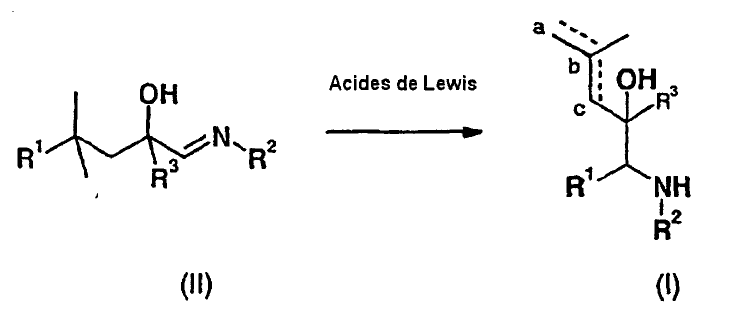 dans laquelle R¹, R² et R³ ont les significations indiquées dans la revendication 1, avec un acide de Lewis dans un solvant organique et on obtient des composés de formule générale I après une purification usuelle.
